(19) Europäisches Patentamt / European Patent Office / Office européen des brevets

(11) **EP 3 782 591 B1**

(12) **EUROPEAN PATENT SPECIFICATION**

(45) Date of publication and mention
of the grant of the patent:
**29.11.2023 Bulletin 2023/48**

(21) Application number: **20201021.1**

(22) Date of filing: **07.07.2017**

(51) International Patent Classification (IPC):
***A61F 9/008*** (2006.01)

(52) Cooperative Patent Classification (CPC):
**A61F 9/00827; A61F 9/00829;** A61F 2009/00872;
A61F 2009/0088; A61F 2009/00882

(54) **LENTICULAR LASER INCISION USING WAVEFRONT GUIDED MAPS**

LINSENFÖRMIGER LASEREINSCHNITT UNTER VERWENDUNG VON
WELLENFRONTGEFÜHRTEN KARTEN

INCISION LASER LENTICULAIRE UTILISANT DES CARTES GUIDÉES DE FRONT D'ONDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.07.2016 US 201662359634 P**

(43) Date of publication of application:
**24.02.2021 Bulletin 2021/08**

(62) Document number(s) of the earlier application(s) in
accordance with Art. 76 EPC:
**17740270.8 / 3 481 346**

(73) Proprietor: **AMO Development, LLC
Santa Ana, CA 92705 (US)**

(72) Inventors:
• **FU, Hong
Santa Ana, CA California 92705 (US)**
• **MALEK TABRIZI, Alireza
Santa Ana, CA California 92705 (US)**
• **CHERNYAK, Dimitri
Sunnyvale, CA California 94087 (US)**
• **DAI, Guangming G.
Santa Ana, CA California 92705 (US)**
• **NEAL, Daniel R.
Santa Ana, CA California 92705 (US)**
• **RAYMOND, Thomas D.
Santa Ana, CA California 92705 (US)**

(74) Representative: **Carpmaels & Ransford LLP
One Southampton Row
London WC1B 5HA (GB)**

(56) References cited:
**WO-A1-2011/035063 WO-A1-2016/100439
US-A1- 2016 089 270**

**Description**

**FIELD**

Embodiments of this invention relate generally to laser-assisted ophthalmic systems for making lenticular incisions in the cornea based on wavefront-guided measurements.

**BACKGROUND**

Vision impairments such as myopia (near-sightedness), hyperopia and astigmatism can be corrected using eyeglasses or contact lenses. Alternatively, the cornea of the eye can be reshaped surgically to provide the needed optical correction. Eye surgery has become commonplace with some patients pursuing it as an elective procedure to avoid using contact lenses or glasses to correct refractive problems, and others pursuing it to correct adverse conditions such as cataracts. And, with recent developments in laser technology, laser surgery is becoming the technique of choice for ophthalmic procedures. The reason eye surgeons prefer a surgical laser beam over manual tools like microkeratomes and forceps is that the laser beam can be focused precisely on extremely small amounts of ocular tissue, thereby enhancing accuracy and reliability of the procedure. These in turn enable better wound healing and recovery following surgery.

Hyperopia (far-sightedness) is a visual impairment where light entering the eye does not focus at the retina to produce a sharp image as desired, but rather focuses at a location behind the retina such that a patient sees a blurred disc. The basic principle to treating hyperopia is to add positive focusing power to the cornea. For instance, a hyperopic eye can be treated by placing a convex lens in front of the eye to add a positive focusing power to the eye. After correction, light passing through the convex lens and into the eye focuses at the retina to form a sharp image.

Different laser eye surgical systems use different types of laser beams for the various procedures and indications. These include, for instance, ultraviolet lasers, infrared lasers, and near-infrared, ultra-short pulsed lasers. Ultra-short pulsed lasers emit radiation with pulse durations as short as 10 femtoseconds and as long as 3 nanoseconds, and a wavelength between 300 nm and 3000 nm. Examples of laser systems that provide ultra-short pulsed laser beams include the Abbott Medical Optics iFS Advanced Femtosecond Laser, the IntraLase FS Laser, and OptiMedica's Catalys Precision Laser System.

Prior surgical approaches for reshaping the cornea include laser assisted *in situ* keratomileusis (hereinafter "LASIK"), photorefractive keratectomy (hereinafter "PRK") and Small Incision Lens Extraction (hereinafter "SMILE").

In the LASIK procedure, an ultra-short pulsed laser is used to cut a corneal flap to expose the corneal stroma for photoablation with ultraviolet beams from an excimer laser. Photoablation of the corneal stroma reshapes the cornea and corrects the refractive condition such as myopia, hyperopia, astigmatism, and the like. A device useful in such a procedure is disclosed in WO2011/035036, which uses a wavefront aberrometer to measure the corneal profile. Subsequently, a first imager images the free eye and a second imager images an applanated eye. The images obtained are compared and the shape of a corneal flap incision is adapted accordingly.

It is known that if part of the cornea is removed, the pressure exerted on the cornea by the aqueous humor in the anterior chamber of the eye will act to close the void created in the cornea, resulting in a reshaped cornea. By properly selecting the size, shape and location of a corneal void, one can obtain the desired shape, and hence, the desired optical properties of the cornea.

In current laser surgery treatments that correct hyperopia using LASIK and PRK, positive focusing power is added to the cornea by steepening the curvature of the cornea, by for example, removing a ring-shaped stroma material from the cornea. In a LASIK procedure, a flap is created, and then lifted up so that the ring-shaped stroma material can be removed or ablated away by an excimer laser. The center of the cornea is not removed while more outward portions of the cornea are removed. The flap is then put back into place. The cornea thus steepens due to the void created in the cornea. Common patterns that steepen the cornea include ring, tunnel and toric shapes. LASIK can typically correct hyperopia for up to 5D (diopter). In a PRK procedure, no flap is created. Instead, an excimer laser is used to first remove the epithelium layer and then the ring-shaped stroma material. The epithelium layer grows back a few days following the procedure.

More recently, surgeons have started using another surgical technique called small incision lenticule extraction (SMILE) for refractive correction. The SMILE procedure is different from LASIK and PRK. Instead of ablating corneal tissue with an excimer laser, the SMILE technique involves tissue removal with two femtosecond laser incisions that intersect to create a lenticule, which is then extracted. Lenticular extractions can be performed either with or without the creation of a corneal flap. With the flapless procedure, a refractive lenticule is created in the intact portion of the anterior cornea and removed through a small incision.

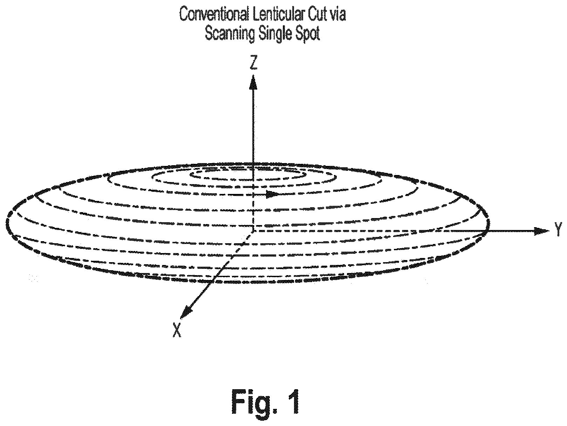

Further, as shown in **FIG. 1,** conventional femtosecond laser surgery systems generate a curved dissection surface to make a lenticular incision by scanning a laser focus on the intended dissection surface through a XY-scanning device and a Z-scanning device. This method does not use the more advantageous "fast-scan-slow-sweep" scanning scheme with femtosecond lasers having high repetition rate ("rep rate"), for *e.g.,* in the MHz range. Using the "fast-scan-slow-sweep" scanning scheme for a lenticular incision, however, will generate vertical "steps" and will require many vertical side cuts, resulting in a lenticular dissection surface that is not smooth.

Typically, the manifest refraction method was used to measure the eye to calculate the necessary correction of aberrations. Such measurements, however, may have inherent disadvantages in that the error rate can be high, higher order aberrations are not measured and/or corrected for, and with manifest refraction, no objective reference exists for the lateral position of lenticule incisions.

Therefore, there is a need for improved systems and methods for measuring for correction as well as for generating corneal lenticular incisions for vision correction using high repetition rate femtosecond lasers.

## SUMMARY

Hence, to obviate one or more problems due to limitations and disadvantages of the related art, this disclosure provides embodiments including an ophthalmic surgical laser system comprising a laser delivery system for delivering a pulsed laser beam to a target in a subject's eye, an XY-scan device to deflect the pulsed laser beam, a Z-scan device to modify a depth of a focus of the pulsed laser beam, and a controller configured to form a top lenticular incision and a bottom lenticular incision of a lens on the subject's eye. The XY-scan device deflects the pulsed laser beam to form a scan line. The scan line is tangential to the parallels of latitude of the lens. The scan line is then moved along the meridians of longitude of the lens. The top lenticular incision is moved over the top surface of the lens through the apex of the top surface of the lens, and the bottom lenticular incision is moved over the bottom surface of the lens through the apex of bottom surface of the lens.

Other embodiments disclose an ophthalmic surgical laser system comprising a laser delivery system for delivering a pulsed laser beam to a target in a subject's eye, an XY-scan device to deflect the pulsed laser beam, a Z-scan device to modify a depth of a focus of the pulsed laser beam, and a controller configured to form a top concave lenticular incision and a bottom concave lenticular incision of a lens on the subject's eye.

In particular, a system is provided for performing ophthalmic surgery, according to claim 1. The system comprises a wavefront aberrometer, configured to obtain a wavefront map of an eye in its natural state to measure a refractive error, a first iris imager, in communication with the wavefront aberrometer, configured to obtain a first iris image of the eye in its natural state, and a laser surgery device including a patient interface configured to dock to the eye, wherein a portion of the patient interface that contacts the patient cornea comprises a liquid interface, a second iris imager, configured to obtain a second iris image for the docked eye and a computer processor and memory in communication with the wavefront aberrometer, configured to determine a cutting profile to cut in a cornea of the eye in its natural state, based on the wavefront aberrometer measurement; and determine a translation of the cutting profile for the docked eye using the cutting profile, the iris image for the eye in its natural state and the iris image for the docked eye. An ultrashort pulsed laser is in communication with the computer processor. The cutting profile for the eye in its natural state includes a lenticule shape comprising a top lenticular incision and a bottom lenticular incision, and the computer processor and memory are further configured to determine a first translation of the cutting profile based on an estimated perturbation of the eye by docking to the patient interface, and determine a second translation of the cutting profile for the docked eye using the iris image for the docked eye compared to the iris image of the eye in its natural state, wherein the second translation of the cutting profile accounts for iris distortion in the second iris image for the docked eye. The ultrashort pulsed laser is configured to incise the bottom lenticular incision and the top lenticular incision in the cornea on the docked eye based on the first translation of the cutting profile and the second translation of the cutting profile.

## BRIEF DESCRIPTION OF THE DRAWINGS

The novel features of the embodiments are set forth with particularity in the appended claims. A better understanding of the features and advantages will be facilitated by referring to the following detailed description that sets forth illustrative, as well as to the accompanying drawings, in which like numerals refer to like parts throughout the different views. Like parts, however, do not always have like reference numerals. Further, the drawings are not drawn to scale, and emphasis has instead been placed on illustrating the principles of the embodiments. All illustrations are intended to convey concepts, where relative sizes, shapes, and other detailed attributes may be illustrated schematically rather than depicted literally or precisely.

**FIG. 1** illustrates a conventional lenticular cut by scanning a single focus spot.
**FIG. 2** is a simplified diagram of a surgical ophthalmic laser system.
**FIG. 3** is another simplified diagram of a surgical ophthalmic laser system.
**FIG. 4** is a simplified diagram of a controller of a surgical ophthalmic laser system.

**FIG. 5** illustrates an exemplary scanning of a surgical ophthalmic laser system.

**FIG. 6** illustrates an exemplary lenticular incision using a fast-scan-slow-sweep scheme of a surgical ophthalmic laser system.

**FIG. 7** illustrates a geometric relation between a fast scan line and an intended spherical dissection surface of a surgical ophthalmic laser system.

**FIG. 8** illustrates an exemplary lenticular incision using a surgical ophthalmic laser system.

**FIG. 9** is a flowchart illustrating a process according to some embodiments.

**FIG. 10** illustrates an example Small Incision Lenticule Extraction procedure.

**FIG. 11** illustrates another example Small Incision Lenticule Extraction procedure.

**FIG. 12** illustrates an exemplary lenticular incision process.

**FIG. 13** illustrates an exemplary lenticular incision using a surgical ophthalmic laser system.

**FIG. 14** illustrates an exemplary scanning process using a surgical ophthalmic laser system.

**FIG. 15A** is a flowchart illustrating an exemplary surgery process.

**FIG. 15B** shows a diagram explaining some of the steps of FIG. 15A in more detail.

**FIGs. 16A** and **16B** together illustrate an assembly of a suitable configuration and integration of an optical coherence tomography subsystem, a wavefront aberrometry subsystem, a corneal topographer subsystem, an iris imaging subsystem, and a fixation target subsystem.

**FIG. 17** is a 3-dimensional representation of an anterior portion of an eye obtained using the optical measurement system.

**FIG. 18** is a flowchart of an example embodiment of a method for performing cataract diagnostics for an eye with an optical measurement instrument, including wavefront aberrometry, corneal topography, and OCT measurements at various locations with the eye along the axial length of the eye.

**FIG. 19** is a diagram showing side views of corneas and effects of patient interfaces.

**FIG. 20A** is a diagram showing example translations of lenticules from side angles.

**FIG. 20B** is another diagram showing example translations of lenticules from side angles.

## DETAILED DESCRIPTION

Embodiments here are generally directed to systems for laser-assisted ophthalmic procedures, and more particularly, to systems for lenticular laser incisions.

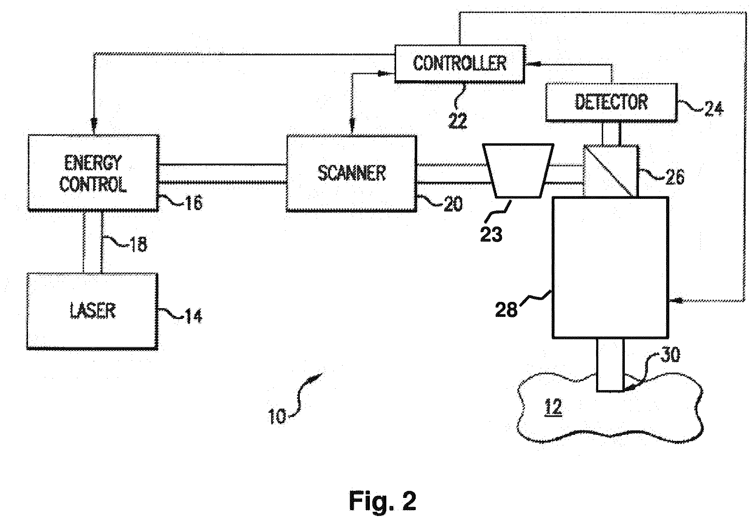

Referring to the drawings, **FIG. 2** shows a system 10 for making an incision in a material 12. The system 10 includes, but is not limited to, a laser 14 capable of generating a pulsed laser beam 18, an energy control module 16 for varying the pulse energy of the pulsed laser beam 18, a Z-scanner 20 for modifying the depth of the pulse laser beam 18, a controller 22, a prism 23 (*e.g.,* a Dove or Pechan prism, or the like), and an XY-scanner 28 for deflecting or directing the pulsed laser beam 18 from the laser 14 on or within the material 12. The controller 22, such as a processor operating suitable control software, is operatively coupled with the Z-scanner 20, the XY-scanner 28, and the energy control unit 16 to direct a scan line 30 of the pulsed laser beam along a scan pattern on or in the material 12. In this embodiment, the system 10 further includes a beam splitter 26 and a detector 24 coupled to the controller 22 for a feedback control mechanism (not shown) of the pulsed laser beam 18. Other feedback methods may also be used, including but not necessarily limited to position encoder on the scanner 20, or the like. In an embodiment, the pattern of pulses may be summarized in machine readable data of tangible storage media in the form of a treatment table. The treatment table may be adjusted according to feedback input into the controller 22 from an automated image analysis system in response to feedback data provided from an ablation monitoring system feedback system (not shown). Optionally, the feedback may be manually entered into the controller 22 by a system operator. The feedback may also be provided by integrating a wavefront measurement system (not shown) with the laser surgery system 10. The controller 22 may continue and/or terminate a sculpting or incision in response to the feedback, and may also modify the planned sculpting or incision based at least in part on the feedback. Measurement and imaging systems are further described in U.S. Patent Nos. 6,315,413 and 8,260,024.

In some embodiments, the system 10 uses a pair of scanning mirrors or other optics (not shown) to angularly deflect and scan the pulsed laser beam 18. For example, scanning mirrors driven by galvanometers may be employed where each of the mirrors scans the pulsed laser beam 18 along one of two orthogonal axes. A focusing objective (not shown), whether one lens or several lenses, images the pulsed laser beam 18 onto a focal plane of the system 10. The focal point of the pulsed laser beam 18 may thus be scanned in two dimensions (*e.g.*, the x-axis and the y-axis) within the focal plane of the system 10. Scanning along the third dimension, *i.e.,* moving the focal plane along an optical axis (*e.g.*, the z-axis), may be achieved by moving the focusing objective, or one or more lenses within the focusing objective, along the optical axis.

Laser 14 may comprise a femtosecond laser capable of providing pulsed laser beams, which may be used in optical procedures, such as localized photodisruption (*e.g.*, laser induced optical breakdown). Localized photodisruptions

4

can be placed at or below the surface of the material to produce high-precision material processing. For example, a micro-optics scanning system may be used to scan the pulsed laser beam to produce an incision in the material, create a flap of the material, create a pocket within the material, form removable structures of the material, and the like. The term "scan" or "scanning" refers to the movement of the focal point of the pulsed laser beam along a desired path or in a desired pattern.

In some embodiments, the laser 14 may comprise a laser source configured to deliver an ultraviolet laser beam comprising a plurality of ultraviolet laser pulses capable of photodecomposing one or more intraocular targets within the eye.

Although the laser system 10 may be used to photoalter a variety of materials (*e.g.*, organic, inorganic, or a combination thereof), in some embodiments, the laser system 10 is suitable for ophthalmic applications. In these cases, the focusing optics direct the pulsed laser beam 18 toward an eye (for example, onto or into a cornea) for plasma mediated (for example, non-UV) photoablation of superficial tissue, or into the stroma of the cornea for intrastromal photodisruption of tissue. In these embodiments, the surgical laser system 10 may also include a lens to change the shape (for example, flatten or curve) of the cornea prior to scanning the pulsed laser beam 18 toward the eye.

The laser system 10 is capable of generating the pulsed laser beam 18 with physical characteristics similar to those of the laser beams generated by a laser system disclosed in U.S. Pat. No. 4,764,930, U.S. Pat. No. 5,993,438, U.S. Patent Application Serial No. 12/987,069, filed January 7, 2011, filed January 7, 2011 (published as US20110172649), U.S. Patent Application Serial No. 13/798,457 filed March 13, 2013 (published as US20140104576), U.S. Patent Application Serial No. 14/848,733, filed September 9, 2015, U.S. Patent Application Serial No. 14/865,396, filed September 25, 2015, U.S. Patent Application Serial No. 14/968,549, filed December 14, 2015, and U.S. Patent Application Serial No. 14/970,898, filed December 16, 2015.

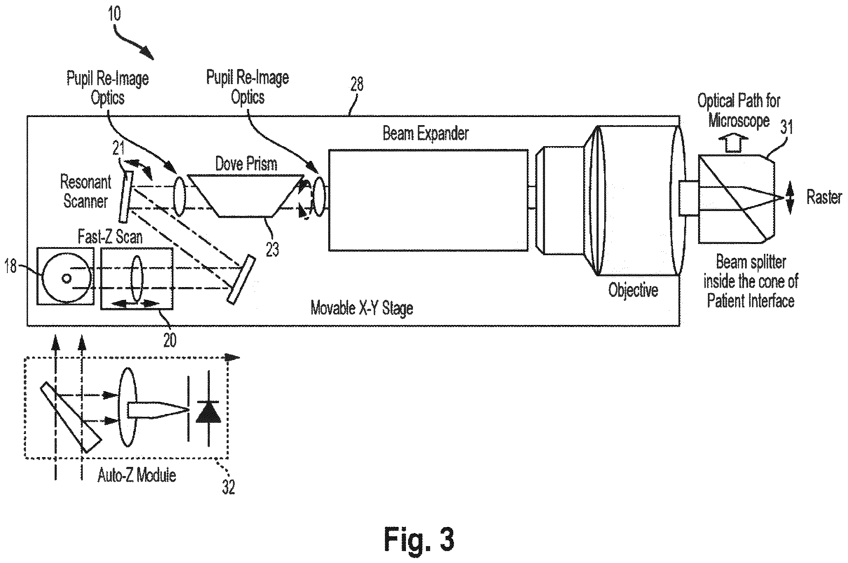

**FIG. 3** shows another exemplary diagram of the laser system 10. **FIG. 3** shows a moveable XY-scanner (or XY-stage) 28 of a miniaturized femtosecond laser system. In this embodiment, the system 10 uses a femtosecond oscillator, or a fiber oscillator-based low energy laser. This allows the laser to be made much smaller. The laser-tissue interaction is in the low-density-plasma mode. An exemplary set of laser parameters for such lasers include pulse energy in the 50-100nJ range and pulse repetitive rates (or "rep rates") in the 5-20MHz range. A fast-Z scanner 20 and a resonant scanner 21 direct the laser beam 18 to the prism 23. When used in an ophthalmic procedure, the system 10 also includes a patient interface 31 design that has a fixed cone nose and a portion that engages with the patient's eye. A beam splitter is placed inside the cone of the patient interface to allow the whole eye to be imaged via visualization optics. In one embodiment, the system 10 uses: optics with a 0.6 numerical aperture (NA) which would produce 1.1 $\mu$m Full Width at Half Maximum (FWHM) focus spot size; and a resonant scanner 21 that produces 1-2 mm scan line with the XY-scanner scanning the resonant scan line to a 10mm field. The prism 23 rotates the resonant scan line in any direction on the XY plane. The fast-Z scanner 20 sets the incision depth and produces a side cut. The system 10 may also include an auto-Z module 32 to provide depth reference. The miniaturized femtosecond laser system 10 may be a desktop system so that the patient sits upright while being under treatment. This eliminates the need of certain opto-mechanical arm mechanism(s), and greatly reduces the complexity, size, and weight of the laser system. Alternatively, the miniaturized laser system may be designed as a conventional femtosecond laser system, where the patient is treated while lying down.

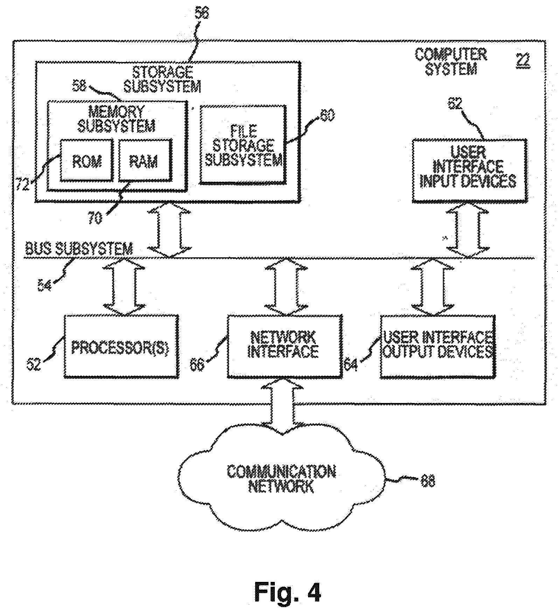

**FIG. 4** illustrates a simplified block diagram of an exemplary controller 22 that may be used by the laser system 10 according to some embodiments. Controller 22 typically includes at least one processor 52 which may communicate with a number of peripheral devices via a bus subsystem 54. These peripheral devices may include a storage subsystem 56, comprising a memory subsystem 58 and a file storage subsystem 60, user interface input devices 62, user interface output devices 64, and a network interface subsystem 66. Network interface subsystem 66 provides an interface to outside networks 68 and/or other devices. Network interface subsystem 66 includes one or more interfaces known in the arts, such as LAN, WLAN, Bluetooth, other wire and wireless interfaces, and so on.

User interface input devices 62 may include a keyboard, pointing devices such as a mouse, trackball, touch pad, or graphics tablet, a scanner, foot pedals, a joystick, a touch screen incorporated into a display, audio input devices such as voice recognition systems, microphones, and other types of input devices. In general, the term "input device" is intended to include a variety of conventional and proprietary devices and ways to input information into controller 22.

User interface output devices 64 may include a display subsystem, a printer, a fax machine, or non-visual displays such as audio output devices. The display subsystem may be a flat-panel device such as a liquid crystal display (LCD), a light emitting diode (LED) display, a touchscreen display, or the like. The display subsystem may also provide a non-visual display such as via audio output devices. In general, the term "output device" is intended to include a variety of conventional and proprietary devices and ways to output information from controller 22 to a user.

Storage subsystem 56 can store the basic programming and data constructs that provide the functionality of the various embodiments. For example, a database and modules implementing the functionality of the present embodiments, as described herein, may be stored in storage subsystem 56. These software modules are generally executed by processor 52. In a distributed environment, the software modules may be stored on a plurality of computer systems and executed by processors of the plurality of computer systems. Storage subsystem 56 typically comprises memory subsystem 58 and file storage subsystem 60.

Memory subsystem 58 typically includes a number of memories including a main random access memory (RAM) 70 for storage of instructions and data during program execution and a read only memory (ROM) 72 in which fixed instructions are stored. File storage subsystem 60 provides persistent (non-volatile) storage for program and data files. File storage subsystem 60 may include a hard disk drive along with associated removable media, a Compact Disk (CD) drive, an optical drive, DVD, solid-state memory, and/or other removable media. One or more of the drives may be located at remote locations on other connected computers at other sites coupled to controller 22. The modules implementing the functionality of the present embodiments may be stored by file storage subsystem 60.

Bus subsystem 54 provides a mechanism for letting the various components and subsystems of controller 22 communicate with each other as intended. The various subsystems and components of controller 22 need not be at the same physical location but may be distributed at various locations within a distributed network. Although bus subsystem 54 is shown schematically as a single bus, alternate embodiments of the bus subsystem may utilize multiple busses.

Due to the ever-changing nature of computers and networks, the description of controller 22 depicted in **FIG. 4** is intended only as an example for purposes of illustrating only some embodiments. Many other configurations of controller 22, having more or fewer components than those depicted in **FIG. 4,** are possible.

As should be understood by those of skill in the art, additional components and subsystems may be included with laser system 10. For example, spatial and/or temporal integrators may be included to control the distribution of energy within the laser beam, as described in U.S. Patent No. 5,646,791. Ablation effluent evacuators/filters, aspirators, and other ancillary components of the surgical laser system are known in the art, and may be included in the system. In addition, an imaging device or system may be used to guide the laser beam. Further details of suitable components of subsystems that can be incorporated into an ophthalmic laser system for performing the procedures described here can be found in commonly-assigned U.S. Patent No. 4,665,913, U.S. Patent No. 4,669,466, U.S. Patent No. 4,732,148, U.S. Patent No. 4,770,172, U.S. Patent No. 4,773,414, U.S. Patent No. 5,207,668, U.S. Patent No. 5,108,388, U.S. Patent No. 5,219,343, U.S. Patent No. 5,646,791, U. S. Patent No. 5,163,934, U.S. Patent No. 8,394,084, U.S. Patent No. 8,403,921, U.S. Patent No. 8,690,862, U.S. Patent No. 8,709,001, U.S. Application Serial No. 12/987,069, filed January 7, 2011, U.S. Patent Application Serial No. 13/798,457 filed March 13, 2013 (published as US20140104576), U.S. Patent Application Serial No. 14/848,733, filed September 9, 2015, U.S. Patent Application Serial No. 14/865,396, filed September 25, 2015, U.S. Patent Application Serial No. 14/968,549, filed December 14, 2015, and U.S. Patent Application Serial No. 14/970,898, filed December 16, 2015.

In one embodiment, the laser surgery system 10 includes a femtosecond oscillator-based laser operating in the MHz range, for example, 10 MHz, for example, from several MHz to tens of MHz. For ophthalmic applications, the XY-scanner 28 may utilize a pair of scanning mirrors or other optics (not shown) to angularly deflect and scan the pulsed laser beam 18. For example, scanning mirrors driven by galvanometers may be employed, each scanning the pulsed laser beam 18 along one of two orthogonal axes. A focusing objective (not shown), whether one lens or several lenses, images the pulsed laser beam onto a focal plane of the laser surgery system 10. The focal point of the pulsed laser beam 18 may thus be scanned in two dimensions (*e.g.*, the X-axis and the Y-axis) within the focal plane of the laser surgery system 10. Scanning along a third dimension, *i.e.,* moving the focal plane along an optical axis (*e.g.,* the Z-axis), may be achieved by moving the focusing objective, or one or more lenses within the focusing objective, along the optical axis. In many embodiments, the XY-scanner 28 deflects the pulse laser beam 18 to form a scan line.

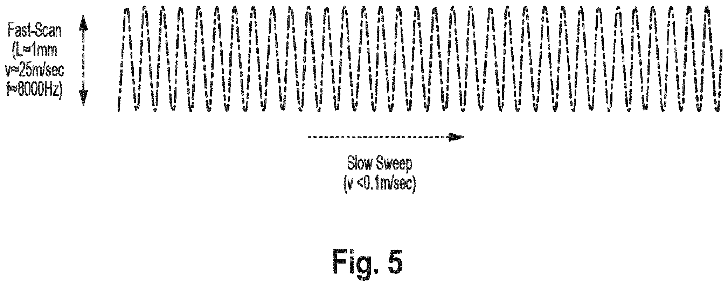

In other embodiments, the beam scanning can be realized with a "fast-scan-slow-sweep" scanning scheme. The scheme consists of two scanning mechanisms: first, a high frequency fast scanner is used to produce a short, fast scan line (*e.g.,* a resonant scanner 21 of **FIG. 3**); second, the fast scan line is slowly swept by much slower X, Y, and Z scan mechanisms. **FIG. 5** illustrates a scanning example of a laser system 10 using an 8 kHz resonant scanner 21 to produce a scan line of about I mm and a scan speed of about 25m/sec, and X, Y, and Z scan mechanisms with the scan speed smaller than 0.1m/sec. The fast scan line may be perpendicular to the optical beam propagation direction, *i.e.,* it is always parallel to the XY plane. The trajectory of the slow sweep can be any three dimensional curve drawn by the X, Y, and Z scanning devices (*e.g.*, XY-scanner 28 and Z-scanner 20). An advantage of the "fast-scan-slow-sweep" scanning scheme is that it only uses small field optics (*e.g.,* a field diameter of 1.5 mm) which can achieve high focus quality at relatively low cost. The large surgical field (e.g., a field diameter of 10 mm or greater) is achieved with the XY-scanner, which may be unlimited.

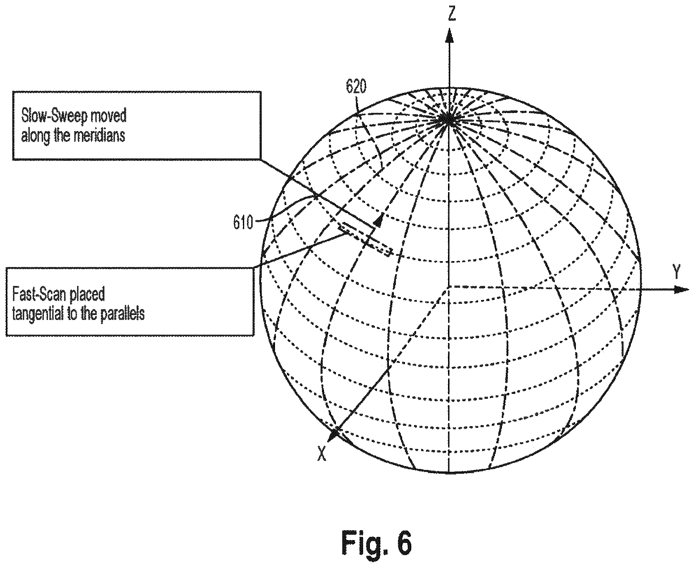

In another embodiment shown in **FIG. 6,** the laser system 10 creates a smooth lenticular cut using the "fast-scan-slow-sweep" scanning scheme under a preferred procedure. First, in a three dimensional lenticular cut, the fast scan line is preferably placed tangential to the parallels of latitude 610. For example, in the miniaturized flap maker laser system 10 of **FIG. 3,** this can be realized by adjusting a prism 23 to the corresponding orientations via software, e.g., via the controller 22. Second, the slow sweep trajectory preferably moves along the meridians of longitude 620. For example, in the miniaturized flap maker system of **FIG. 3,** this can be done by coordinating the XY scanner 28, and the Fast-Z scanner 20 via the software, *e.g.*, via the controller 22. The procedure starts with the scan line being parallel to the XY plane, and sweeps through the apex of the lens, following the curvature with the largest diameter (*see also* **FIG.**

8). With this preferred procedure, there are no vertical "steps" in the dissection, and vertical side cuts are eliminated. As will be analyzed herein below, the deviations between the laser focus locations and the intended spherical surface dissections are also minimized.

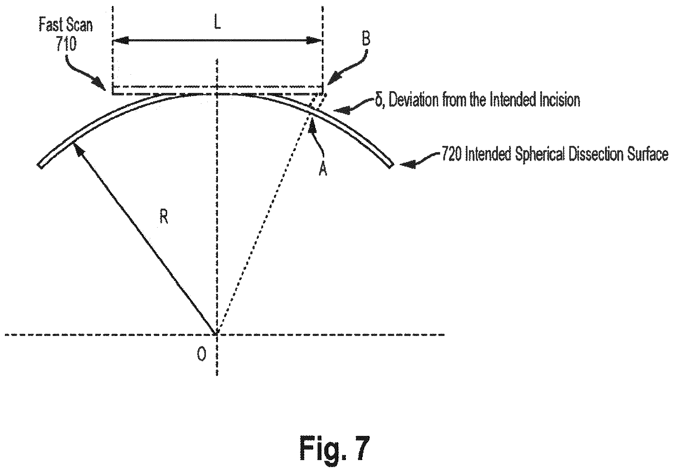

**FIG. 7** shows the geometric relation between the fast scan line 710 and the intended spherical dissection surface 720, *e.g.,* of a lens, especially the distance deviation ($\delta$) between the end point **B** of the scan line 720 and point **A** on the intended dissection surface 720. The maximum deviation $\delta$ is the distance between point **A** and point **B,** and is given by

$$\delta = \sqrt{R^2 + \frac{L^2}{4}} - R \approx \frac{L^2}{8R}$$ , equation (1),

where R is greater than L. R is the radius of curvature of the surface dissection 720, and L is the length of the fast scan.

In an exemplary case of myopic correction, the radius of curvature of the surface dissection may be determined by the amount of correction, $\Delta D$, using the following equation

$$\Delta D = \frac{(n-1)}{R_1} + \frac{(n-1)}{R_2}$$ , equation (2),

where n = 1.376, which is the refractive index of cornea, and $R_1$ and $R_2$ (may also be referred herein as $R_t$ and $R_b$) are the radii of curvature for the top surface and bottom surface of a lenticular incision, respectively. For a lenticular incision with $R_1 = R_2 = R$ (the two dissection surface are equal for them to physically match and be in contact), we have

$$R = \frac{2(n-1)}{\Delta D}$$ , equation (3).

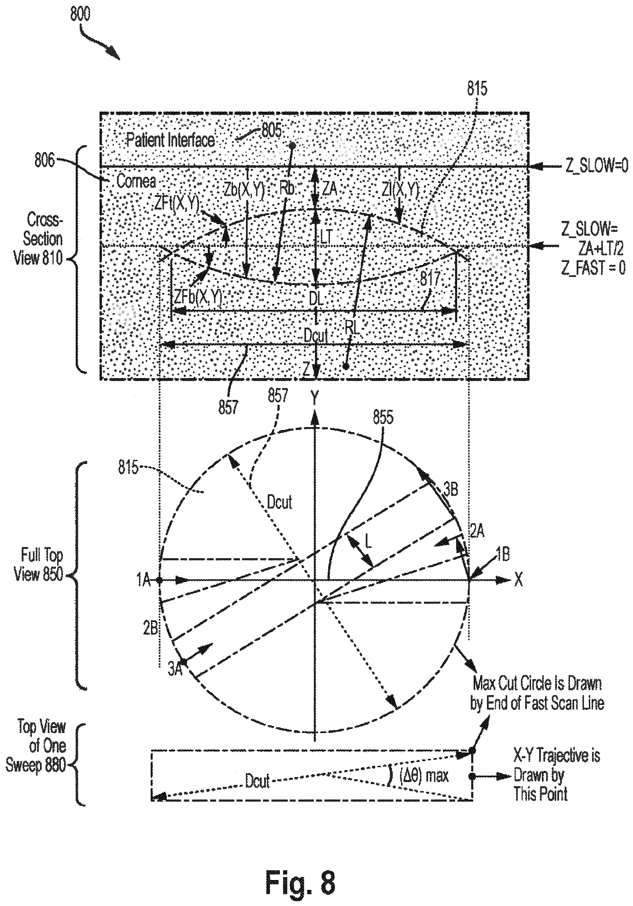

In an embodiment, **FIG. 8** shows an exemplary lenticular incision 800 for extraction using the laser system 10. **FIG. 8** shows an exemplary cross-sectional view 810 illustrating a patient interface 805 (or patient interface 31 as shown in **FIG. 3**), cornea 806, and lenticular incision volume 815, which will be referred herein as lens to be extracted. Rt and Rb are the radii of curvature for the top surface and bottom surface of a lenticular incision, respectively. ZFt (Zt) is the depth of the top surface of the lenticular incision. ZFb (Zb) is the depth of the bottom surface of the lenticular incision. The Z depths may be calculated based on the respective radii. LT is the lens thickness at the lens apex, or center thickness of the lens. ZA is depth of the lens apex. DL is the diameter of the lenticular incision, or the lens. {Z_SLOW = 0} is the Z reference position before the laser system 10 calculates and sets Z_SLOW, *e.g.,* {Z_SLOW = ZA + LT/2} the center depth of the lens, which remains fixed for the duration of the incision procedure. Z_SLOW may then be the reference position for the Z-scanner for top and bottom incision surfaces. In an embodiment, the diameter of the lens may be received from an operator of the laser system 10, or may be calculated by the laser system 10. The thickness of the lens may be determined, for example, by the total amount of correction (*e.g.*, diopter) and the diameter of the lens.

A top view 850 of the lenticular incision 800 illustrates three exemplary sweeps (1A to 1B), (2A to 2B) and (3A to 3B), with each sweep going through (*i.e.*, going over) the lenticular incision apex 855. The incision, or cut, diameter 857 ($D_{CUT}$) should be equal to or greater than the to-be-extracted lenticular incision diameter 817 (DL). A top view 880 shows the top view of one exemplary sweep. In an embodiment, the lenticular incision is performed using the following steps:

1. Calculate the radius of curvature based on the amount of correction, *e.g.,* a myopic correction.
2. Select the diameter for the lenticular incision to be extracted.
3. Perform the side incision first (not shown) to provide a vent for gas that can be produced in the lenticular surface dissections. This is also the incision for the entry of forceps and for lens extraction.
4. Perform bottom surface dissection (the lower dissection as shown in cross-sectional view 810). In doing so, the fast scan line is preferably kept tangential to the parallels of latitude, and the trajectory of the slow sweep drawn by X, Y, and Z scanning devices moves along the meridians of longitude (near south pole in a sequence of IA →1B (first sweep of lenticular cut), 2A → 2B (second sweep of lenticular cut), 3A → 3B (third sweep of lenticular cut), and so on, until the full bottom dissection surface is generated.

5. Perform the top surface dissection (the upper dissection as shown in the cross-sectional view 810) in a similar manner as the bottom dissection is done. The bottom dissection must be performed first. Otherwise, the bubble generated during the top dissection will block the laser beam from making the bottom dissection.

For illustrative purposes, in a myopic correction of $\Delta D$ = 10 diopter (*i.e.,* 1/m), using equation (3), R = 75.2mm, which is indeed much greater than the length L of the fast scan. Assuming a reasonable scan line length of L = 1mm, using equation (1), the deviation $\delta \approx 1.7$ $\mu$m. This deviation is thus very small. For comparison purpose, the depth of focus of a one micron (FWHM) spot size at 1$\mu$m wavelength is about $\pm 3$ $\mu$m, meaning the length of focus is greater than the deviation $\delta$.

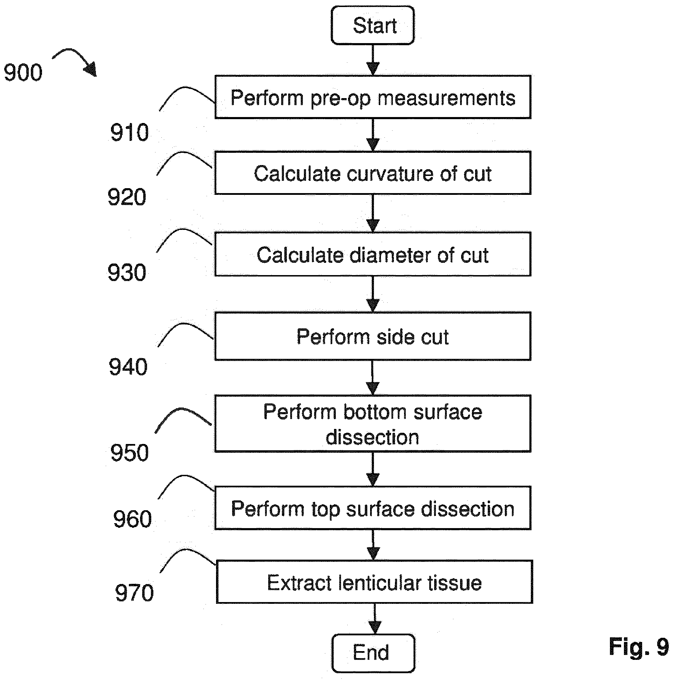

**FIG. 9** illustrates a process 900 of the laser system 10 according to an embodiment. The laser system 10 may start a surgical procedure performing pre-operation measurements 910. For example, in an ophthalmologic surgery for myopic correction, the myopic diopter is determined, the SLOW_Z position is determined, and so on. The laser system 10 calculates the radius of curvature based on the amount of correction, *e.g.,* the myopic correction determined in pre-operation measurements 920, as shown, for example, in equations (2) and (3) above. The laser system 10 calculates the diameter of the incision 930, as shown by $D_{CUT}$ in **FIG. 8**. $D_{CUT}$ is equal to or greater than the diameter of the to-be-extracted lenticule (DL in **FIG. 8**). The laser system 10 first performs side incision to provide a vent for gas that can be produced in the lenticular surface dissections, and for tissue extraction later on 940. The laser system 10 then performs the bottom lenticular surface dissection 950 before performing the top lenticular surface dissection 960. The lenticular tissue is then extracted 970.

In other embodiments, the laser system 10 may also be used to produce other three-dimensional surface shapes, including toric surfaces for correcting hyperopia and astigmatism. The laser system 10 may also be used for laser material processing and micromachining for other transparent materials. Correction of hyperopia by the laser system 10 is discussed in detail below.

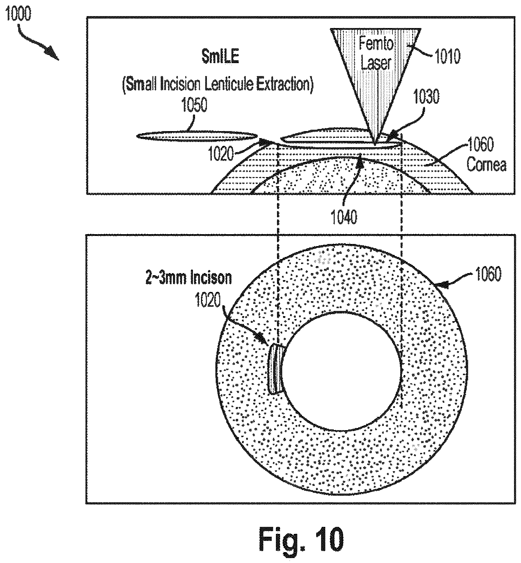

Conventional laser surgery methods to correct hyperopia utilize cut patterns including ring-shaped incision patterns that steepen the curvature of a cornea. In the SMILE procedure illustrated in **FIG. 10,** a femtolaser 1010 is used to make a side cut 1020, an upper surface cut 1030 and a lower surface cut 1040 that forms a cut lens 1050. A manual extraction tool such as a tweezer is then used to extract the cut lens beneath the anterior surface of the cornea 1060 through the side cut 1020. Recently, SMILE has been applied to treat myopia by cutting and extracting a convex lens-shaped stroma material with a femtosecond laser. However, SMILE techniques have not been applied in treating hyperopia.

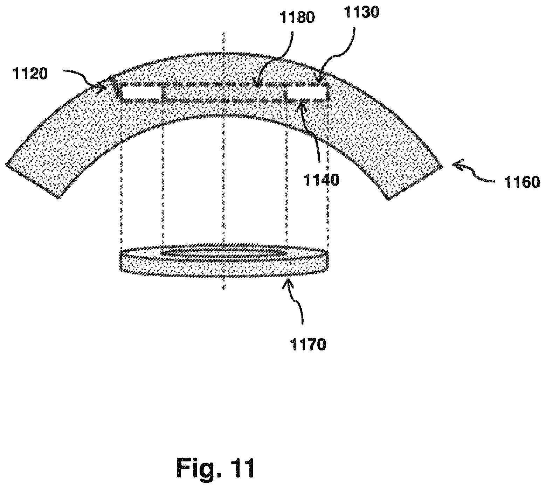

However, **FIG. 11** illustrates why utilizing these patterns using SMILE is impractical and unfeasible. The cross-sectional view of the cornea 1160 in **FIG. 11** includes a side cut 1120, an upper surface cut 1130, lower surface cut 1140 and a ring-shaped cut 170 generated by a SMILE procedure. However, the cornea 1160 maintains an uncut annular center portion 180 that remains attached to an anterior portion and posterior portion of the cornea 1160.

This cut pattern is geometrically problematic as the clean removal of the ring cut 1170 through the side cut 1120 as a single ring is impeded by the center portion 1180. Whereas a flap provided in a LASIK procedure allows a ring shape to be easily extracted, the use of a side cut without a flap prevents the ring-shaped stroma material from being extracted from the tunnel like incision without breaking apart. Thus, a ring-shaped lenticule is not suitable for correcting hyperopia using the SMILE procedure since the ring cut 1170 will break up unpredictably during removal through the side cut 1120.

Some LASIK procedures correct hyperopia by removing cornea stroma material to increase the steepness of the cornea. For example, outward portions of the cornea are cut and removed while a center portion remains untouched except for the flap. Once the flap is folded back over, the flap fills the void vacated by the removed cornea stroma material and merges with the cornea. The cornea thus becomes steeper and a desired vision correction is achieved. However, the curve of the flap does not match the curve of the cornea such that the merger of the flap and cornea creates folds in the stroma that increase light scattering and create undesirable aberrations.

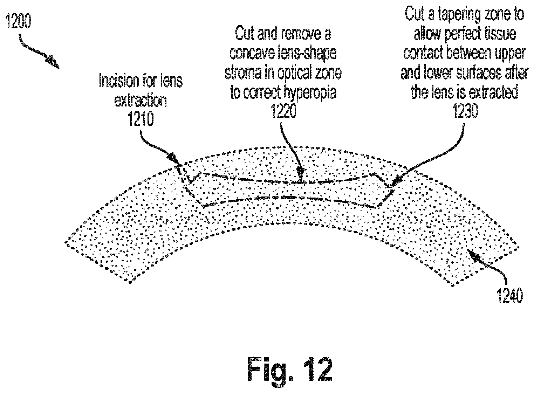

The embodiments described herein overcome these limitations. **FIG. 12** illustrates an exemplary lenticular incision 1200 that steepens the cornea by cutting and removing a symmetric concave lens-shaped stroma material from a cornea 1240. From an optical focus power perspective, the concave shape of the lenticule 1220 is equivalent to steepening the cornea or adding a convex lens in front of the eye.

Furthermore, extraction of the lenticule 1220 as a whole piece through a side cut incision 1210 is assured and improved over a ring-shape cut, or a tunnel-like cut, or a toric cut. The incision includes a peripheral portion 1230 or tapering portion providing ideal merging of the cornea after the lenticule 1220 is extracted without folding in a top surface or bottom surface.

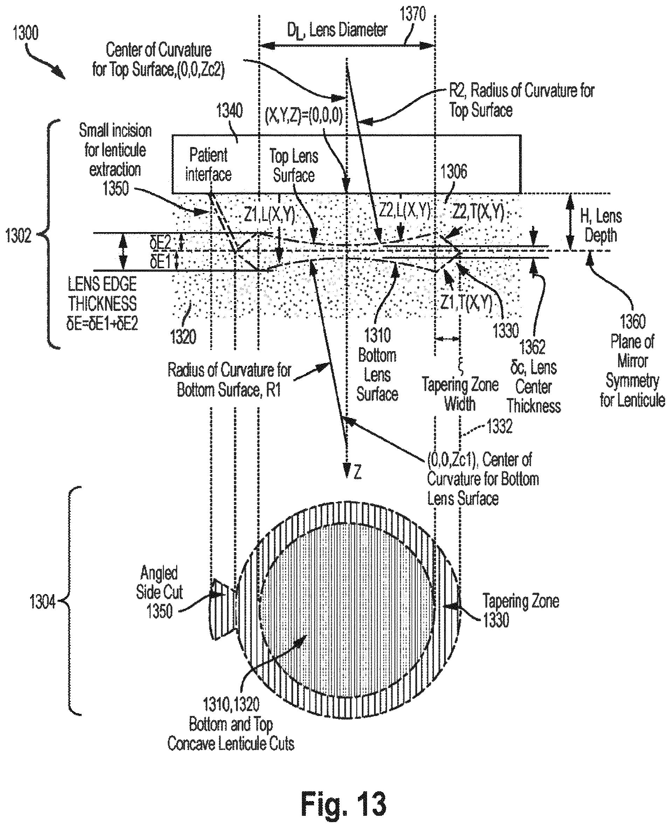

**FIG. 13** illustrates an exemplary lenticular incision 1300 using a surgical ophthalmic laser system according to some embodiments. For example, SMILE techniques may be applied in conjunction with **FIG. 13** to treat hyperopia using a sub-nanosecond laser. A cross-sectional view 1302 and top view 1304 are provided of the lenticule cuts 1310, 1320 and side cut 1350. In **FIG. 13,** a patient interface 1340 is pressed against a cornea 1306. The lenticular incision includes a bottom lens surface 1310 and a top lens surface 1320. The bottom surface 1310 includes a radius of curvature R1 and the top surface 1320 includes a radius of curvature R2.

A side cut 1350 is performed first to provide a path for gas to vent to prevent the formation of bubbles. A bottom surface cut 1310 is then performed prior to performing a top surface cut 1320 to prevent the cutting beam from being blocked by bubbles generated by previous cornea dissection. The top and bottom surface cuts each include a central portion and a peripheral portion. The central portions are concave while the peripheral portions of the top and bottom cuts tapers (diminishes) towards each other to meet in a circumferential ring or edge. The tapering peripheral portions minimize light scattering at the edges and further optimizes the matching of the cut surfaces and prevent folding after the lenticule has been removed.

As shown in **FIG. 13,** the thickest portion of the cut is provided at the boundary of the taper portion and the concave portion. For the top and bottom surfaces to match after lens extraction, the bottom and top surfaces are preferably mirror symmetric about a plane 1360.

These exemplary lenticular incisions allow lenticular tissue to be extracted in a single unbroken piece through the side cut. The taper of the peripheral portions allows smooth extraction through the side cut as a gradual slope is provided. The peripheral portions also support the merging of the top and bottom portions of the cornea as a top surface and bottom surface compress back together to form a smooth merge. Without a taper to the peripheral portions, the apex of the central portions would never merge and would form a permanent gap.

A concave lens cut includes a top concave lenticular incision and a bottom concave lenticular incision of a lens in the subject's eye. The concave lens cut may include at least one of a spherical surface, a cylindrical component, and any high order component. The top concave lenticular incision and the bottom concave lenticular incision may be mirror symmetric or nearly mirror symmetric to each other so long as the merging of the top surface and bottom surface does not create folding.

The system may operate with a laser having a wavelength in a range between 350 nanometers and 1100 nanometers, and a pulse width in a range between 10 femtoseconds and 1 nanosecond.

In prior art solutions, a top layer cut is longer than a bottom layer cut. Under this configuration, the top and bottom cornea portions do not ideally merge as the top surface must fold in and compress in order to merge with shorter layer cut. With this fold created by the dissection, light scattering is increased. In contrast, a mirror symmetric cut along a center line allows ideal merge with no folding between a top layer and bottom layer. Consequently, there is less light scattering.

A lens edge thickness is given by $\delta_E$, $\delta_{E1}$, $\delta_{E2}$. A lens depth H is given as a distance between an anterior of the cornea 1306 and the plane 1360. The bottom surface 1310 and top surface 1320 have a lens diameter $D_L$, a lens center thickness $\delta c$ and a shape defined by respective curves $Z_{1,L}(x,y)$ and $Z_{2,L}(x,y)$. In order to minimize the amount of dissected cornea stroma material removed, the central thickness $\delta c$ should be minimized. For example, the central thickness may be a few $\mu$m, which can be achieved by using a laser beam with a high numerical aperture (such as NA = 0.6).

Each of the bottom lens surface cut 1310 and the top lens surface cut 1320 includes a tapering zone 1330 along a periphery of the cuts. The tapering zone 1330 is defined by a tapering zone width $\xi$ and the curves $Z_{1,T}(x,y)$ and $Z_{2,T}(x,y)$.

A side cut 1350 is provided from a surface of the cornea to the tapering zone 1330 for removal of the lenticule. The side cut may meet the tapering zone 1330 on the mirror plane 1360 or other suitable extraction point.

With these parameters as described and illustrated, a set of equations are provided below that determine the three-dimensional shape of the lenticular cuts, assuming that the desired correction is purely defocus:

$$Z_{1,L}(x,y) = H + \frac{\delta_c}{2} + R_1 - \sqrt{R_1^2 - x^2 - y^2} \qquad \text{for} \qquad \sqrt{x^2 + y^2} \leq \frac{D_L}{2}$$

Eq.(4)

$$Z_{2,L}(x,y) = H - \frac{\delta_c}{2} - R_2 + \sqrt{R_2^2 - x^2 - y^2} \qquad \text{for} \qquad \sqrt{x^2 + y^2} \leq \frac{D_L}{2}$$

Eq.(5)

$$Z_{1,T}(x,y) = H + \delta_{E1} - \left(\sqrt{x^2 + y^2} - \frac{D_L}{2}\right) \cdot \frac{\delta_{E1}}{\xi} \quad \text{for} \quad \frac{D_L}{2} \leq \sqrt{x^2 + y^2} \leq \frac{D_L}{2} + \xi$$

Eq.(6)

$$Z_{2,T}(x,y) = H - \delta_{E2} + \left(\sqrt{x^2 + y^2} - \frac{D_L}{2}\right) \cdot \frac{\delta_{E2}}{\xi} \quad \text{for} \quad \frac{D_L}{2} \leq \sqrt{x^2 + y^2} \leq \frac{D_L}{2} + \xi$$

Eq.(7)

$$\delta_{E1} = \frac{\delta_C}{2} + R_1 - \sqrt{R_1^2 - \left(\frac{D_L}{2}\right)^2} \qquad \text{Eq.(8)}$$

$$\delta_{E2} = \frac{\delta_C}{2} + R_2 - \sqrt{R_2^2 - \left(\frac{D_L}{2}\right)^2} \qquad \text{Eq.(9)}$$

The shape and dimensions of the cuts may include additional correction for higher order aberrations and may be computed from measured vision errors. In some embodiments, approximately 50% of the total hyperopic correction is applied to each of the two mutually mirror-imaged cut surfaces.

It is noted that the thickest portion of the concave lens cut is provided at the intersection of the tapering zone and the concave lens cuts which correspond to a portion of the cornea that is thicker than a center portion of the cornea. Consequently, from the standpoint of cornea thickness, correcting hyperopia is more tolerable than correcting myopia, where the thicker portion of the lens to be removed is at the center of the cornea, corresponding to a thinner portion of the cornea.

The shape of the tapering zone 1330 need not be linear in shape. The tapering zone may be curved or any shape that minimizes light scattering at the cutting junctions and optimizes the matching of the two cut surfaces after lens extraction. The peripheral zone may be linear or a higher order polynomial.

Some embodiments apply to single-spot scanning methods applied in femtosecond laser systems. The embodiments also apply to cornea incisions using UV 355 nm sub-nanosecond lasers.

For illustrative purposes, Equations (2), (8) and (9) are used to estimate the thickness of the concave lens. In a hyperopic correction of $\Delta D = 5$ diopter (which is high end values for LASIK hyperopia procedures) and assuming that a symmetric shape of the lenticule is selected, $R_1 = R_2 = 150.4$ mm. Assuming $D_L = 7.0$ mm and $\delta_C = 10$ $\mu$m, then $\delta_E = \delta_{E1} + \delta_{E2} \approx \delta_C + D_L^2 \cdot \Delta D/[8(n-1)] \approx 92 \mu$m.

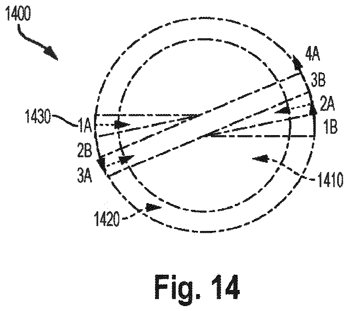

**FIG. 14** illustrates an exemplary scanning process 1400 using a surgical ophthalmic laser system according to some embodiments. **FIG. 14** illustrates another embodiment of the "Fast-Scan-Slow-Sweep" scanning described previously. While performing an XY scan, Z values can be calculated from Eqs.(1)-(9), and the desired three-dimensional concave lens-shape cutting surfaces may be generated.

A top view of the lenticular incision illustrates three exemplary sweeps 1430 (1A to 1B), (2A to 2B) and (3A to 3B), with each sweep going through (*i.e.,* going over) the concave lenticular incision 1410 and tapering zone 1420. In an embodiment, the lenticular incision is performed in the following steps:

1. Calculate the radius of curvature based on the amount of correction, *e.g.,* a hyperopic correction.
2. Select the diameter for the lenticular incision to be extracted.
3. Calculate the shape of the lenticular incisions (concave surface and taper).
4. Perform the side incision first (not shown) to provide a vent for gas that can be produced in the lenticular surface dissections. This is also the incision for the entry of forceps and for lens extraction.
5. Perform bottom surface dissection (the bottom dissection 1310 as shown in cross-sectional view). In doing so, the fast scan line is preferably kept tangential to the parallels of latitude, and the trajectory of the slow sweep drawn by X, Y, and Z scanning devices moves along the meridians of longitude (near south pole in a sequence of IA →1B (first sweep of lenticular cut), 2A → 2B (second sweep of lenticular cut), 3A → 3B (third sweep of lenticular cut), and so on (4A), until the full bottom dissection surface is generated.
6. Perform the top surface dissection 1320 in a similar manner as the bottom dissection is done. It is noted that the bottom dissection is done first. Otherwise, the bubble generated during the top dissection will block the laser beam in making the bottom dissection.

**Wavefront Map Guided Lenticular Incisions**

Previously, manifest refraction measurements were used to determine the femtosecond laser parameters to form a lenticule in a cornea. However, such measurements may have inherent disadvantages. For example, error may be large, the higher order aberrations may not be measured and therefore not corrected for.

Using some other measurement, such as a wavefront map of a patient's eye which measures aberrations of light that pass through the cornea and lens, a lenticular shape may be determined. Such wavefront guided measurements may be more precise, up to +/-0.01D, and higher order aberrations may be measured and corrected. Iris imaging may be used for positioning/aligning the laser system to the eye to account for such things as cyclorotation when a patient lies in a supine position for treatment. Iris imaging may also be used for patient registration and recognition.

In a high level example, the system may be used to measure a pupil diameter to calculate a theoretical perfect eye map with the same pupil size and also capture an image of the iris.

Next, the system may capture the round shave wavefront from the eye, the light reflected from the retina and after back through the pupil.

Next, the system may map any captured aberrations and compare them to the theoretical perfect eye map. The comparison may be used to determine corrections that need to be made and that information may be used to incise a lenticule in the patient cornea with the femtosecond pulsed lasers described here.

**Aberrations**

Old manifest refraction could measure and correct for low order aberrations such as sphere and cylinder which may be corrected for using glasses. But there are many higher-order aberrations including Zernike coefficients, circle aberrations, spherical aberration, coma and trefoil. The wavefront guided systems here may correct for some or all of these higher order aberrations as they may be measured by the wavefront system.

For example, spherical aberration is the cause of dark condition myopia and may result in the patient experiencing visualized halos around lights. In light conditions, peripheral rays may be blocked when the pupil constricts, lessening the halo effect. In dark conditions, peripheral rays may not be blocked when the pupil is enlarged causing a slight myopia. Other aberrations include Coma and Trefoil.

These higher order aberrations may be detected, analyzed and used to determine a treatment using the systems and methods described here. Other aberrations such as asymmetrical aberrations may also be measured and corrected using the systems and methods here. For example, a previous off-center surgery may result in an eye with asymmetrical correction. Using the systems and methods here, that asymmetry may be measured and an asymmetrical lenticule may be incised to compensate.

Certain example embodiments may be used to not remove every aberration but to strategically leave certain aberrations to compensate for other optical corrections that are not otherwise correctable. On top of the wavefront error, certain things may be added back to accomplish a goal. For example, to extend depth of focus, you may wish to leave a circular aberration so that a person with presbyopia, a condition where the lens of the eye is no longer able to adjust for near focus, can minimally compromise their far sight but also correct for near sight.

**Steps to Use Wavefront Guided Lenticular Incisions**

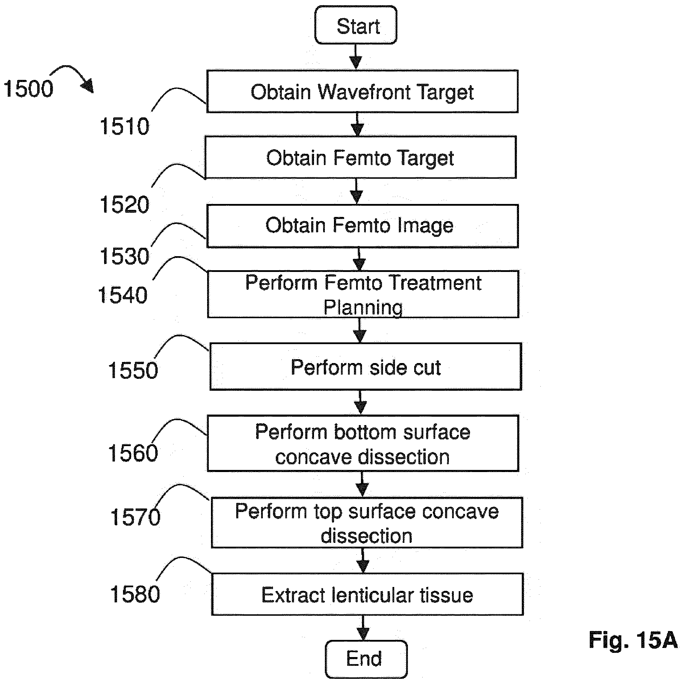

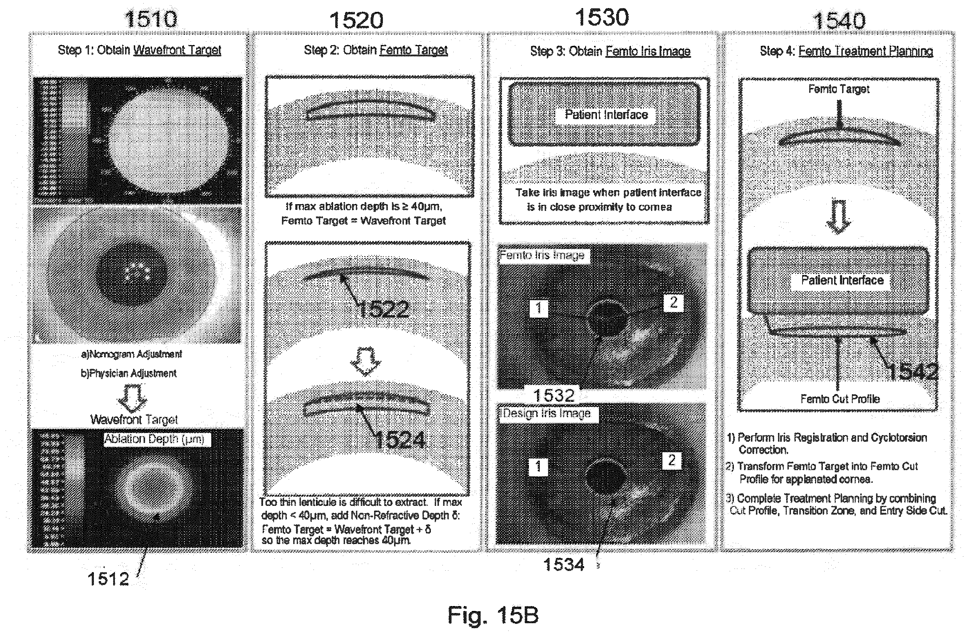

FIG. 15A shows example steps that may be used in obtaining and using a wavefront map of an eye to form a treatment plan using a femtosecond laser as described herein. FIG. 15B shows diagrams explaining the first four steps in more detail according to some embodiments.

Step-1510: Obtain Wavefront Target. In this step, a wavefront map is measured of an eye and an iris image is taken for the eye under its natural, non-accommodative, far vision condition. Nomogram adjustment and physician adjustment may be applied to determine the ablation depth map 1512, or Wavefront Target, which is to be removed from the cornea to achieve the desired vision correction.

Step-1520: Obtain Femto Target. Since a too thin lenticule 1522 may be difficult to extract from a cornea, it may be useful to add a constant, non-refractive depth 1524 to the determined Wavefront Target, if its maximum depth is less than, $40\mu m$, for example. And, if the Wavefront Target is too thick so the residual stroma bed is less than $250\mu m$, a reduction in thickness may be made by reducing the diameter of the optical zone and removing a constant, non-refractive depth from the Wavefront Target. The thickness adjusted (or intact) target may be referred to as the Femto Target, which is the desired tissue shape to remove from the cornea. It should be noted that non-refractive depth, are not defined by two concentric spherical surfaces. Rather, it is a small, constant z-displacement for the anterior cornea surface in the optical zone, so the cornea shape and refractive power remain unchanged after the displacement.

Step-1530: Obtain Femto Iris Image. Next, when the patient is gazing at the fixation light and when the patient interface is in close proximity (but not in contact) to the cornea, an iris image may be made. This image 1532, 1534 may be used to map areas of the iris for alignment purposes and/or identification purposes. Note: the deformation of eye under applanation will be taking into account by Step-1540.

Step-1540: Perform Femto Treatment Planning which may include several sub-steps. A) Perform Iris Registration and Cyclotorsion Correction for alignment purposes. B) Transform Femto Target to Femto Cut Profile. The shape of a lenticule in cornea under applanation is different from that in a free cornea, therefore, the Femto Target may be transformed into a Femto Cut Profile for the cornea shape when the system is docked to it 1542. C) Complete Femto Treatment Planning to integrate Femto Cut Profile, Transition Zone to taper the lenticule for extraction, Entry Side Cut, and possibly another Internal Side Cut, to achieve optimum precision and accuracy of the lenticule shape, easy lenticule release, minimum total laser pulse energy, and shorter cutting time.

The laser system 10 may be used to perform a side incision to provide a vent for gas that can be produced in the lenticular surface dissections, and for tissue extraction later on 1550. The laser system 10 then performs the bottom lenticular surface dissection 1560 before performing the top lenticular surface dissection 1570. Performing the dissections in this order allows gas to vent out of the cornea instead of becoming trapped in gas bubbles within the cornea. The lenticular tissue may then be extracted 1580.

**Wavefront Aberrometer Systems**

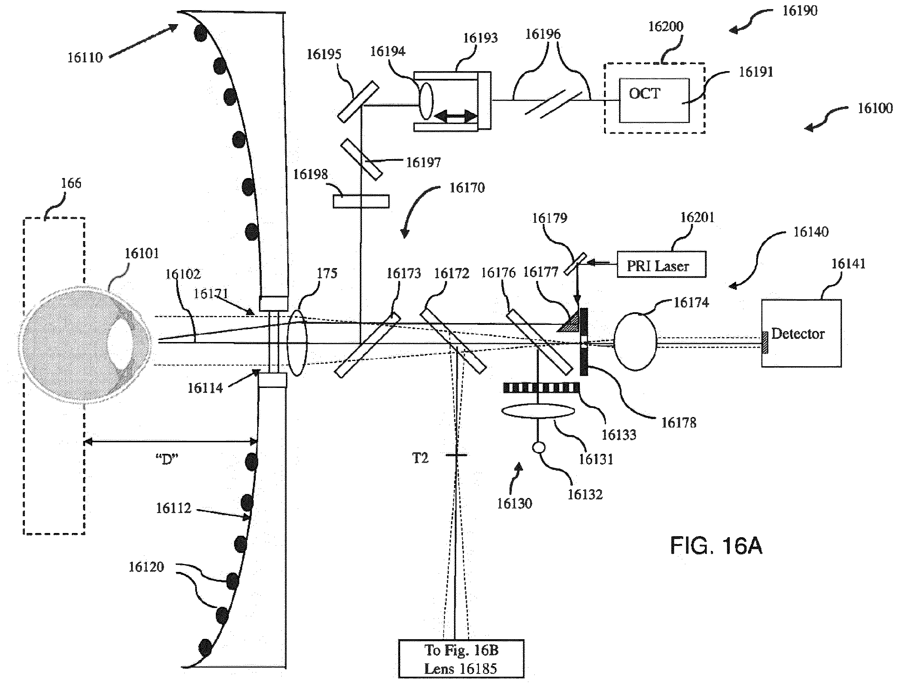

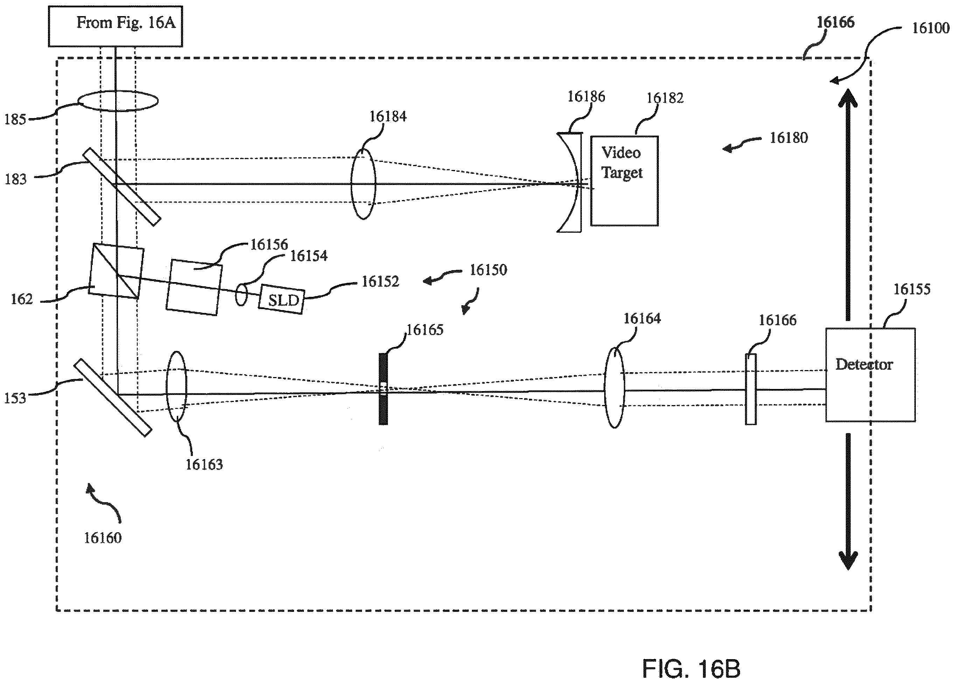

FIGs. 16A and 16B shows an example wavefront aberrometer which may be used to take the measurements as described here. The assembly in FIG. 16A and 16B 16100 is a nonlimiting example of suitable configurations and integration of not only the wavefront aberrometer subsystem 16150, but the optical coherence tomographer (OCT) subsystem 16190, the corneal topographer subsystem 16140 for measuring one or more characteristics of a subject's eye, an iris imaging subsystem 1640, the fixation target subsystem 16180 and the shared optics.

The wavefront aberrometer subsystem 16150 of the assembly 16100 includes a light source 16152 providing a probe beam and a wavefront sensor 16155. The Wavefront aberrometer subsystem 16150 preferably further comprises a collimating lens 16154, a polarizing beam splitter 16156, an adjustable telescope comprising a first optical element, lens 16163 and a second optical element, lens 16164, a movable stage or platform 16166, and a dynamic-range limiting aperture 16165 for limiting a dynamic range of light provided to wavefront sensor 16155 so as to preclude data ambiguity. Light from the wavefront aberrometer subsystem may be directed to one of the constituent optical elements of the optical system 16170 disposed along a central axis 16102 passing through the opening or aperture 16114 of the structure 16110. It will be appreciated by those of skill in the art that the lenses 16163, 16164, or any of the other lenses discussed herein, may be replaced or supplemented by another type of converging or diverging optical element, such as a diffractive optical element.

Light source 16152 is preferably an 840 nm SLD (super luminescent laser diode). An SLD is similar to a laser in that the light originates from a very small emitter area. However, unlike a laser, the spectral width of the SLD is very broad, about 40 nm. This tends to reduce speckle effects and improve the images that are used for wavefront measurements.

Preferably, wavefront sensor 16155 is a Shack-Hartmann wavefront sensor comprising a detector array and a plurality of lenslets for focusing received light onto its detector array. In that case, the detector array may be a CCD, a CMOS array, or another electronic photosensitive device. However, other wavefront sensors may be employed instead. Embodiments of wavefront sensors which may be employed in one or more systems described herein are described in U.S. Pat. No. 6,550,917, issued to Neal et al. on Apr. 22, 2003, and U.S. Pat. No. 5,777,719, issued to Williams et al. on Jul. 7, 1998.

The aperture or opening in the middle of the group of first light sources 16120 (e.g., aperture 16114 in principal surface 16112 of structure 16110) allows system 16100 to provide a probe beam into eye 16101 to characterize its total ocular aberrations. Accordingly, third light source 16152 supplies a probe beam through a light source polarizing beam splitter 16156 and polarizing beam splitter 16162 to first beam splitter 16172 of optical system 16170. First beam splitter 16172 directs the probe beam through aperture 16114 to eye 16101. Preferably, light from the probe beam is scattered from the retina of eye 16100, and at least a portion of the scattered light passes back through aperture 16114 to first beam splitter 16172. First beam splitter 16172 directs the back scattered light back through beam splitter 16172 to polarizing beam splitter 16162, mirror 16153 to wavefront sensor 16155.

Wavefront sensor 16155 outputs signals to a processor of controller 60 which uses the signals to determine ocular aberrations of eye 16101. Preferably, processor 16141 is able to better characterize eye 16101 by considering the corneal topography of eye 16101 measured by the corneal topography subsystem, which may also be determined by processor 16141 based on outputs of detector array 16141, as explained above.

In operation of the wavefront aberrometer subsystem 16150, light from light source 16152 is collimated by lens 16154. In polarization embodiments, the light passes through light source polarizing beam splitter 16156. The light entering light source polarizing beam splitter 16156 is partially polarized. Light source polarizing beam splitter 16156 reflects light having a first, S, polarization, and transmits light having a second, P, polarization so the exiting light is 100% linearly polarized. In this case, S and P refer to polarization directions relative to the hypotenuse in light source polarizing beam splitter 16156.

Light from light source polarizing beam splitter 16156 enters polarizing beam splitter 16162. The hypotenuse of polarizing beam splitter 16162 is rotated 90 degrees relative to the hypotenuse of light source polarizing beam splitter 16156 so the light is now S polarized relative the hypotenuse of polarizing beam splitter 16162 and therefore the light reflects upwards. The light from polarizing beam splitter 16162 travels upward and passes through toward beam splitter 16172, retaining its S polarization, and then travels through quarter wave plate 16171. Quarter wave plate 16171 converts the light to circular polarization. The light then travels through aperture 16114 in principal surface 16112 of structure 16110 to eye 16101. Preferably, the beam diameter on the cornea is between 1 and 2 mm. Then the light travels through the cornea and focuses onto the retina of eye 16100.

The focused spot of light becomes a light source that is used to characterize eye 16100 with wavefront sensor 16155. Light from the probe beam that impinges on the retina of eye 101 scatters in various directions. Some of the light reflects back as a semi-collimated beam back towards assembly 16100. Upon scattering, about 90% of the light retains its polarization. So the light traveling back towards assembly is substantially still circularly polarized. The light then travels through aperture 16114 in principal surface 16112 of structure 16110, through quarterwave plate 16171, and is converted back to linear polarization. Quarterwave plate 16171 converts the polarization of the light from the eye's retina so that it is P polarized, in contrast to probe beam received from third light source 16150 having the S polarization. This P polarized light then reflects off of first beam splitter 16172, and then reaches polarizing beam splitter 16162. Since the light is now P polarized relative the hypotenuse of polarizing beam splitter 16162, the beam is transmitted and then continues onto mirror 16153. After being reflected by mirror 16153, light is sent to an adjustable telescope comprising a first optical element 16164 and a second optical element (e.g., lens) 16163 and a movable stage or platform 16166. The beam is also directed through a dynamic-range limiting aperture 16165 for limiting a dynamic range of light provided to wavefront sensor 16155 so as to preclude data ambiguity.

When wavefront sensor 16155 is a Shack-Hartmann sensor, the light is collected by the lenslet array in wavefront sensor 16155 and an image of spots appears on the detector array (e.g., CCD) in wavefront sensor 16155. This image is then provided to a process of the controller 60 and analyzed to compute the refraction and aberrations of eye 16101.

### Iris Imaging

The optical measurement systems preferably include an iris imaging subsystem 40 used to properly align the system during measurements and treatment. The imaging subsystem 40 generally comprises an infrared light source, preferably infrared light source 152, and detector 141. In operation light from the light source 152 is directed along second optical path 160 to first optical path 170 and is subsequently directed to eye 101 as described above. Light reflected from the iris of eye 101 is reflected back along first optical path 170 to detector 141. In normal use, an operator will adjust a position or alignment of system 100 in XY and Z directions to align the patient according to the image detector array 141. In one embodiment of the iris imaging subsystem, eye 101 is illuminated with infrared light from light source 152. In this way, the wavefront obtained by wavefront sensor 155 will be registered to the image from detector array 141.

The image that the operator sees is the iris of eye 100. The cornea generally magnifies and slightly displaces the image from the physical location of the iris. So the alignment that is done is actually to the entrance pupil of the eye. This is generally the desired condition for wavefront sensing and iris registration.

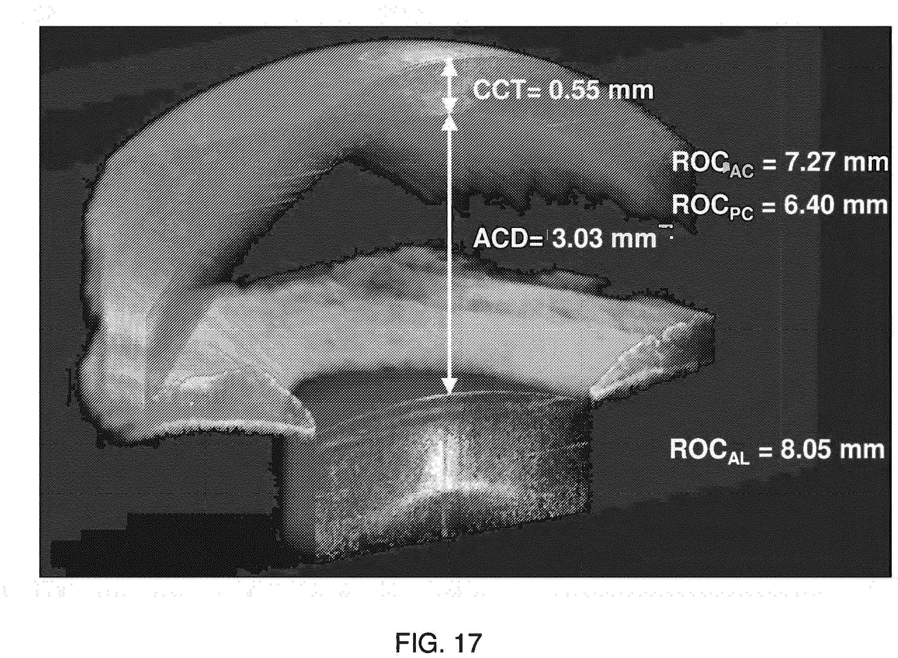

FIG. 17 shows a three-dimensional view of an eye obtained by an optical measurement system using an OCT subsystem according to some embodiments. Fig. 17 evidences that the OCT subsystem of some embodiments is operable to obtain biometry measurements according to some embodiments, including the central corneal thickness (CCT), the anterior chamber depth (ACD), the radius of curvature of the anterior cornea ($ROC_{AC}$), the radius of curvature of the Posterior cornea ($ROC_{PC}$) and the Radius of curvature of the axial length ($ROC_{AL}$).

Preferably, the OCT subsystem 190 provides sufficiently resolved structural information to provide a structural assessment that may provide a user with an indication of suitability of a particular patient for a laser cataract procedure. In some embodiments, an OCT scan performed by the OCT subsystem 190 at or near the retina (i.e., a retina scan) is sufficiently resolved to identify the foveal pit location and depth, wherein a lack of depression indicates an unhealthy retina.

In some embodiments, the optical measurement instrument provides one or more measurements sufficient to provide an assessment of the tear film of a patient. In one embodiment, the tear film assessment comprises a comparison of a wavefront aberrometry map and a corneal topography map or OCT map of the patient's eye, by, for instance, determining the irregular features in either the wavefront aberrometery, and/or corneal topography maps. This can be achieved by first fitting the surface (either wavefront or topography) to smooth functions such as Zernike or Taylor polynomials, and then subtracting this smooth surface from the original surface data. The resulting map is the residual of what does not fit a smooth surface and is highly correlated with the tear film (Haixia Liu, Larry Thibos, Carolyn G. Begley, Arthur Bradley, "MEASUREMENT OF THE TIME COURSE OF OPTICAL QUALITY AND VISUAL DETERIORATION DURING TEAR BREAK-UP," Investigative Ophthalmology & Visual Science, June 2010, Vol. 51, No. 6) . A determination of whether the tear film is broken (if not smooth); an assessment of the tear film, including tear film breakup, can also be obtained by reviewing the shape of spots on the topographer. For instance, a finding or indication that the tear film is disrupted, or broken, may be based upon the shape of a spot in that, if the spots are not round, and have, for instance, an oblong or broken up shape, it indicates that tear film is disrupted. The existence of such a disrupted tear film may indicate that K value, and other ocular measurements may not be reliable. Further indications of the state of the tear film may be made by comparing the OCT and the topographer or wavefront data (Kob - Simultaneous Measurement of Tear Film Dynamics IOVS, July 2010, Vol. 51, No. 7).

In operation, as shown in Fig. 16A, after exiting connector 212, the OCT beam 214 is collimated, preferably using a collimating optical fiber 196. Following collimating fiber 196 the OCT beam 214 is directed to an z-scan device 193 operable to change the focal point of the OCT beam in a z-direction, and x- and y-scan devices 195 and 197, which are operable to scan the OCT beam in x and y-directions perpendicular to the z-direction.

Following the collimating optical fiber 196, the OCT beam 214 continues through a z-scan device 193, 194. Preferably, the z-scan device is a Z telescope 193, which is operable to scan focus position of the laser pulse beam 66 in the patient's eye 101 along the Z axis. For example, the Z-telescope can include a Galilean telescope with two lens groups (each lens group includes one or more lenses). One of the lens groups moves along the Z axis about the collimation position of the Z-telescope 193. In this way, the focus position in the patient's eye 101 moves along the Z axis. In general, there is a relationship between the motion of lens group and the motion of the focus point. The exact relationship between the motion of the lens and the motion of the focus in the z axis of the eye coordinate system does not have to be a fixed linear relationship. The motion can be nonlinear and directed via a model or a calibration from measurement or a combination of both. Alternatively, the other lens group can be moved along the Z axis to adjust the position of the focus point along the Z axis. The Z-telescope 84 functions as a z-scan device for changing the focus point of the OCT beam 214 in the patient's eye 101. The Z-scan device can be controlled automatically and dynamically by the controller 60 and selected to be independent or to interplay with the X and Y scan devices described next.

After passing through the z-scan device, the OCT beam 214 is incident upon an X-scan device 195, which is operable to scan the OCT beam 214 in the X direction, which is dominantly transverse to the Z axis and transverse to the direction of propagation of the OCT beam 214. The X-scan device 195 is controlled by the controller 60, and can include suitable components, such as a lens coupled to a MEMS device, a motor, galvanometer, or any other well-known optic moving device. The relationship of the motion of the beam as a function of the motion of the X actuator does not have to be fixed or linear. Modeling or calibrated measurement of the relationship or a combination of both can be determined and used to direct the location of the beam.

After being directed by the X-scan device 196, the OCT beam 214 is incident upon a Y scan device 197, which is operable to scan the OCT beam 214 in the Y direction, which is dominantly transverse to the X and Z axes. The Y-scan device 197 is controlled by the controller 60, and can include suitable components, such as a lens coupled to a MEMS device, motor, galvanometer, or any other well-known optic moving device. The relationship of the motion of the beam as a function of the motion of the Y actuator does not have to be fixed or linear. Modeling or calibrated measurement of the relationship or a combination of both can be determined and used to direct the location of the beam. Alternatively, the functionality of the X-Scan device 195 and the Y-Scan device 197 can be provided by an XY-scan device configured to scan the laser pulse beam 66 in two dimensions transverse to the Z axis and the propagation direction of the laser pulse beam 66. The X-scan and Y scan devices 195, 197 change the resulting direction of the OCT beam 214, causing lateral displacements of OCT beam 214 located in the patient's eye 101.

The OCT sample beam 214 is then directed to beam splitter 173 through lens 175 through quarter wave plate 171 and aperture 114 and to the patient eye 101. Reflections and scatter off of structures within the eye provide return beams that retrace back through the patient interface quarter wave plate 171, lens 175, beam splitter 173, y-scan device 197, x-scan device 195, z-scan device 193, optical fiber 196 and beam combiner 204 (FIG. 16), and back into the OCT detection device 220. The returning back reflections of the sample arm 201 are combined with the returning reference portion 206 and directed into the detector portion of the OCT detection device 220, which generates OCT signals in response to the combined returning beams. The generated OCT signals that are in turn interpreted by the controller 60 to determine the spatial disposition of the structures of interest in the patient's eye 101. The generated OCT signals can also be interpreted by the controller to determine the spatial disposition of the structures of interest in the patient's eye 101. The generated OCT signals can also be interpreted by the control electronics to align the position and orientation of the patient eye within the patient interface. As the OCT information can be obtained relatively rapidly (B-scans at 200-500 scans per second) this can be used to provide tracking information to the patient alignment system. That is, the center offset of the corneal vertex can be obtained in x, y and z by determining the highest point in the x and y-slices, and then determining an offset from the desired alignment point. The z value is the difference between the highest corneal point and the desired z location. This information can be fed to an XYZ tracker that aligns the systems either by moving the instrument, the patient's head, or internal mirrors and optical elements in the instrument.

The quarter wave plate 171 described above has the effect that light returning into the instrument will have its polarization rotated by ninety degrees relative to the outgoing polarization. This can result in a situation that the OCT reference beam and signal light incident on the detector 220 will have nearly orthogonal polarizations so that the interference signal generated is extremely weak. One effective method to maximize the signal strength is to set the relevant OCT reference and sample light beams to be linearly polarized with, for example, a polarizing controller in both the sample arm and the reference arm. In one such embodiment, a first set 198 of polarization controllers (FIG. 16A), for example a set of polarization rotating fiber paddle adjusters on the OCT source light output, set the polarization of the incident light on the beam splitter 173 to be linearly polarized on that surface. Further, a second set 218 of polarization controllers, such as another set of rotating fiber paddle adjusters, are placed in the reference fiber path leading to the detector 220. Adjustment of the polarization controllers, such as the fiber paddles, will maximize the signal when the reference and signal polarizations match. This allows the system to retain the benefits of having the quarter wave plate 171 for the wavefront sensing portion of the instrument while having minimal impact on the OCT signal strength.

The quarter wave plate 171 may be zero order design at either the OCT wavelength, the wavefront sensor wavelength, or an intermediate wavelength. Practical zero order wave plates made of crossed crystalline quartz plates are low cost and will behave as nearly as ideal over the wavelength range of interests, for instance if the center wavefront sensor wavelength is 840 nm and the center OCT wavelength is 1060 nm. Other alternatives are polymer waveplates or the more expensive achromatic quarter wave plates.

The optical measurement systems preferably comprise an iris imaging subsystem 40. The imaging subsystem 40 generally comprises an infrared light source, preferably infrared light source 152, and detector 141. In operation light from the light source 152 is directed along second optical path 160 to first optical path 170 and is subsequently directed to eye 101 as described above. Light reflected from the iris of eye 101 is reflected back along first optical path 170 to detector 141. In normal use, an operator will adjust a position or alignment of system 100 in XY and Z directions to align the patient according to the image detector array 141. In one embodiment of the iris imaging subsystem, eye 101 is illuminated with infrared light from light source 152. In this way, the wavefront obtained by wavefront sensor 155 will be registered to the image from detector array 141.

The image that the operator sees is the iris of eye 100. The cornea generally magnifies and slightly displaces the image from the physical location of the iris. So the alignment that is done is actually to the entrance pupil of the eye. This is generally the desired condition for wavefront sensing and iris registration.

Iris images obtained by the iris imaging subsystem may be used for registering and/or fusing the multiple data sets obtained by the various subsystems, by methods described for instance in "Method for registering multiple data sets," U.S. Patent Appl. No. No. 12/418,841. As set forth in U.S. Patent Appl. No. 12/418,841, wavefront aberrometry may be fused with corneal topography, optical coherence tomography and wavefront, optical coherence tomography and topography, pachymetry and wavefront, etc. For instance, with image recognition techniques it is possible to find the position and extent of various features in an image. Regarding iris registration images, features that are available include the position, size and shape of the pupil, the position, size and shape of the outer iris boundary (OIB), salient iris features (landmarks) and other features as are determined to be needed. Using these techniques, patient eye movement in between measurements as well as that during a measurement sequence can be identified. Further, changes in the eye itself (including those induced by the measurement, such as changes in the size of the pupil, changes in pupil location, etc.) can be identified.

In certain embodiments, an optical measurement system according the present includes a target fixation subsystem, and an assembly 100 shown in Figures 16A and 16B includes fixation target subsystem 180 which includes a fixation target 182 for the patient to view. Fixation target subsystem 180 may be used to control the patient's accommodation, because it is often desired to measure the refraction and wavefront aberrations when eye 100 is focused at its far point (e.g., because LASIK treatments are primarily based on this). Cylindrical correction and liquid lenses for the target path may also be used. In the target fixation subsystem, a projection of a target, for instance a cross-hair pattern is projected onto the eye of the patient, the cross hair pattern being formed by a backlit LED and a film. In some embodiments, a video target is provided that allows the projection of letters, charts, pictures and/or movies. Certain methods to control accommodation may be to provide the patient with a task "click a button each time you recognize a real word" or "click a button each time the target includes the color purple" in order to insure that the subject is really looking and concentrating on the target.

In operation, light originates from the light source 152 or, alternatively, from video target backlight 182 and lens 186. Lens 185 collects the light and forms an aerial image T2. This aerial image is the one that the patient views. The patient focus is maintained on aerial image 182 during measurement so as to maintain the eye in a fixed focal position.

The operating sequence the optical measurement system and methods is not particularly limited. A scan of the patient's eye may comprise one or more of a wavefront aberrometry measurement of a patient's eye utilizing the wavefront aberrometry subsystem, a corneal topography measurement of a patient's eye and an OCT scan of the patient's eye using the OCT subsystem, wherein the OCT scan includes a scan at each or one or more locations within the eye of the patient. These locations of the OCT scan may correspond to the location of the cornea, the location of the anterior portion of the lens, the location of the posterior portion of the lens and the location of the retina. In a preferred embodiment, the operating sequence includes each of a wavefront aberrometry measurement, a corneal topography measurement and an OCT scan, wherein the OCT scan is taken at least at the retina, the cornea and one of anterior portion of the patient's lens. Preferably, an iris image is taken simultaneously with or sequentially with an each of measurements taken with wavefront aberrometry subsystem the corneal topography subsystem and the OCT subsystem, including an iris image take simultaneously with or sequentially with the location of each OCT scan. This results in improved accuracy in the 3-dimensional modeling of the patient's eye by permitting the various data sets to be fused and merged into a 3-dimensional model.

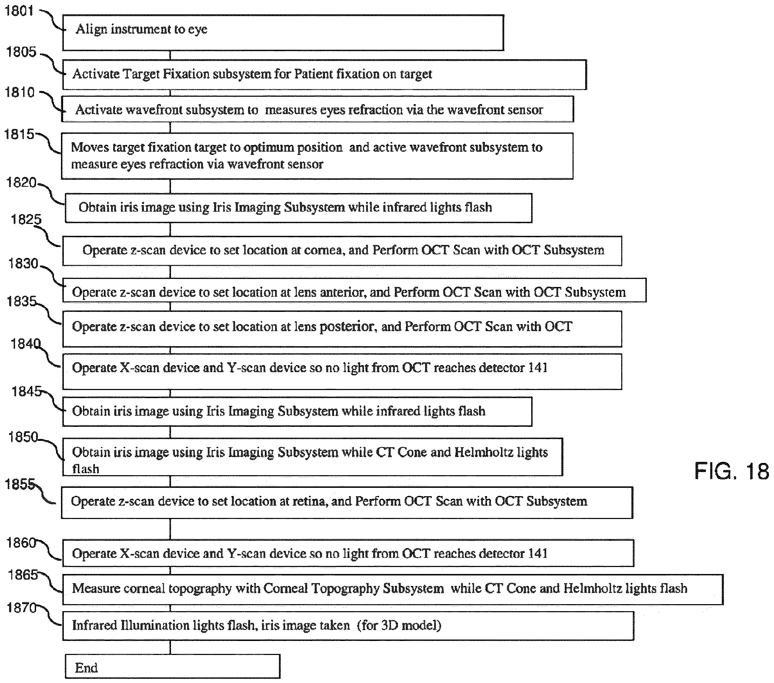

**FIG. 18** shows an example embodiment of an operating sequence and method in which wavefront aberrometry measurements, corneal topography measurements and OCT measurements are all taken. The optical measurement apparatus, including the method of FIG. 18 may be used preoperatively, intra-operatively and/or postoperatively. In the method of FIG. 18, a step 1801 comprises aligning the optical measurement system to the eye of the patent. A step 1805 comprises activating the Target Fixation subsystem for patient fixation on target. A step 1810 comprises activating the wavefront aberrometer subsystem such that the wavefront aberrometer light source 1810 is activated and the eye refraction is measured via the wavefront sensor. A step 1815 comprises activating the target fixation system to move the target to an optimum position and activate the wavefront aberrometer subsystem such that the wavefront aberrometer light source 152 is activated and the eye refraction is measured via the wavefront sensor 155. A step 1820 comprises obtaining an iris image using Iris Imaging Subsystem while infrared light source 152 is operating. A step 1825 comprises operating the z-scan device to set OCT scan location at or near cornea, and performing an OCT Scan with the OCT Subsystem. A step 1830 comprises operating the z-scan device to set the OCT location at a location at or near the lens anterior and performing an OCT Scan with the OCT Subsystem. A step 1835 comprises operating the z-scan device to set the OCT location at a location at or near the lens posterior and performing an OCT Scan with the OCT Subsystem. A step 1840 comprises operating the X-scan device and Y-scan device so no light from OCT reaches detector 141. A step 1845 comprises obtaining an iris image using the Iris Imaging Subsystem while the infrared light source 152 flashes. A step 1850 comprises obtaining an iris image using the Iris Imaging Subsystem while the light sources 120 and Helmholtz source flash. A step 1850 comprises measuring the corneal topography with the Corneal Topography Subsystem. A step 1855 comprises operating the z-scan device to set the OCT location at a location at or near the retina and performing an OCT Scan with the OCT Subsystem. A step 1860 comprises operating the X-scan device and Y-scan device so no light from OCT reaches detector 141. An optional step 1865 comprises measure corneal topography with Corneal Topography Subsystem, which may provide for an improved 3D model of the patient eye. An optional step 1870 comprises obtaining an iris image using Iris Imaging Subsystem (for 3D model).

Using the wavefront map obtained above from the wavefront aberrometer, the femtosecond laser system could be used to incise precise lenticules in corneas, and correct not only low order but also high order aberrations. The combination of these treatment methods is contemplated here. Whilst no claim is directed to these methods *per se,* the laser system of the invention is capable of being used and is intended to be used in such methods.

## Translation of Refractive Error Correction Treatment from Perturbation of Natural Free State of Eye to Applanated Docked Eye

In some example embodiments, an operator may use the wavefront aberrometer as described above to determine a patient's refractive error in their eye, sometimes including higher order aberrations. Corrections for the patient's refractive error may be determined including making plans to form a lenticule in the cornea, or to incise part of the cornea to correct for the determined refractive error. Iris registration may also take place using the wavefront aberrometer to help with identification and alignment of the laser system on a patient. But, such determinations of corrections and iris registration may be made with the wavefront aberrometer on a free eye, one that is in its natural state, not deformed or touched by any device or interface.

In some embodiments, during a laser treatment, a patient interface is used on the eye to be treated. Such interface may touch, dock or otherwise interact with the cornea of the patient's eye. In other words, the eye may be applanated by the patient interface for treatment. This may include compression of the eye and flattening of the cornea.

Embodiments of patient interfaces may include but are not limited to glass or plastic with a liquid interface. If the patient interface touches and pushes on the eye, this applanation may distort and/or otherwise move the cornea from its natural curved shape into a flat shape or flatter shape to conform with the patient interface. Such applanation may also move, distort and/or otherwise stretch the iris of the eye as well. The iris distortion may result from a mechanical deformation by applanation of the patient interface and an apparent distortion due to the change of shape of the eye in the applanation.

Regarding iris registration, the system may use identified features of an iris as a guide to help line up the corrective incisions. Such systems and methods may use multiple features of the iris and use them as landmarks to compare the eye from its natural state to an applanated state. This is because pressing a spherically shaped cornea to a flat plane or flatter plane, may change the cornea-iris relational positions. By comparing multiple landmarks, the system can calibrate the movement of the landmarks before and after applanation to help determine a translation of the corrective incisions and determine the correct coordinates to incise the applanated eye.

Iris imaging may take place using white light illumination and/or infrared illumination. Each of these illuminations may have advantages and disadvantages, such as infrared illumination reducing iris constriction and white light illumination resulting in a sharper image, color images, and allowing a surgeon to visualize the iris in more natural conditions. For example, white light may refer to undivided broad spectrum of wavelengths of light with wavelengths from 400-700 nm and infrared light is higher than the visible spectrum, for example 850 - 1200 nm. In some examples, the white light may be generated using a combination of three sources of light, for example three light emitting diode (LED) lights, each with a different color of red, green, and blue. In some embodiments, a combination of both kinds of illumination, white and infrared, may be used.

Another consideration which may affect the treatment procedure is the orientation of the patient when the wavefront aberrometer is used to determine refractive error and obtain an iris image, and when the incision procedures are performed. In some embodiments, a patient is sitting or standing upright when the wavefront aberrometer is used, and laying down, supine, when the incision procedures are performed. When this orientation change occurs, the eyes of a patient twist slightly in their sockets, this is known as cyclotorsion rotation. In certain embodiments here, this cyclotorsion rotation may be accounted for in the translation of the cutting profile for any patient by adjusting the cutting points according to a previously measured cyclotorsion rotation or a standard cyclotorsion rotation amount.

This iris image translation can be coupled with translation of the refractive error correction determined from the wavefront aberrometer of the eye in its natural state to an applanated eye. The goal of the translation is to help ensure that when the eye returns to its natural state the incisions provide the intended corrections.

The compression of the cornea and eye may also be factored into the translation of the lenticule incisions. When making translation determinations, between how the corrective lenticule may be shaped in the free natural state eye, and the perturbed docked eye, some assumptions may be made. It may be assumed that the cornea conforms to patient interface during docking. It may also be assumed that the cornea in tissue volume does not change during docking. Using these assumptions, maps may be created of the eye based on the addition of a known perturbation to an eye. This addition may be a theoretically based disturbance to the location of the lenticule in the docked eye. It should be noted that empirical corrections of the calculation based on other perturbations or errors for correction may be added to the calculated values or used to modify the calculated values.

Below are calculations for translating points of a lenticule to be incised while it is in its natural free state and in an applanated state. In some embodiments, these relations describe the primary changes introduced by a flat surface or a curved surface of a patient interface applanation. The relationships may be used or the relationships may be further be developed by empirical experimental relations. Thus, the surgeon user may begin with the below information and build in other changes for customization for patient treatment.

**Flat Patient Interface Examples**

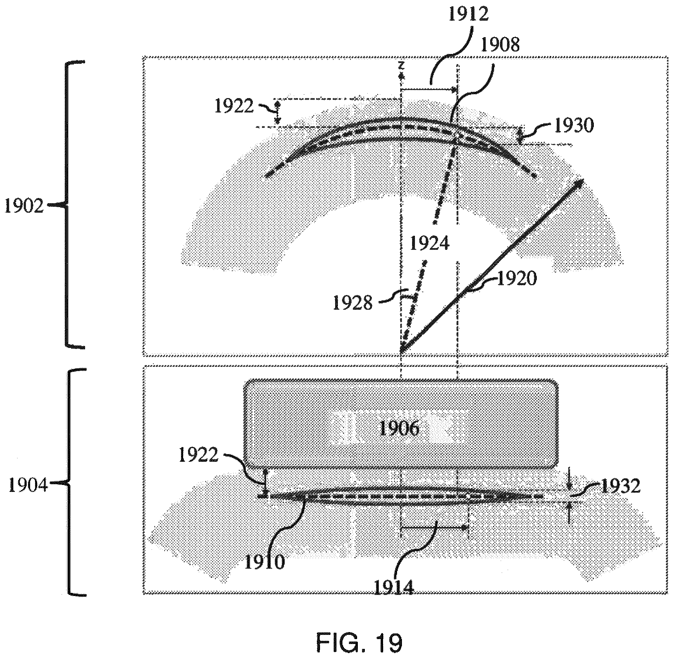

**FIG. 19** shows an example of a two dimensional side view of a cornea in its natural state and 1904 a cornea which is applanated by the flat patient interface 1906. As can be seen, the determined lenticule in the cornea of the natural state eye 1908 is differently shaped than the lenticule when the eye is applanated 1910. The lenticule in the applanated eye 1910 is flattened and wider than the lenticule in the natural state eye 1908. The curve of the two lenticules is different.

An ablation depth profile may be determined to remove the desired lenticule, even if the cornea is applanated. To remove a predetermined lenticule shape from a free cornea, we can transform the known shape into the cutting shape for eye under applanation by either a flat or a curved patient interfaces.

Where 1920 is Ra which is a Radius of curvature of anterior cornea. And 1922 is H which is a cut depth of lenticule central plane. And 1924 is R=Ra-H which is a Radius of curvature of the lenticular central plane, before applanation. And $(\rho, \varphi)$ is a Location of interest before applanation with $\rho$ as 1912. And 1928 is $\theta$ which is an Angle subtended by location of interest relative to Z-axis. And 1930 is $\Delta(\rho, \varphi)$ which is a Z-difference between lenticule surfaces at $(\rho, \varphi)$ before applanation. And 1932 is $(\rho', \varphi)$ which is a location of interest under flat applanation. And $\Delta'(\rho',\varphi)$ is a Z-difference between lenticule surfaces at $(\rho',\varphi)$ under flat applanation with $\rho'$ as 1914.

Thus the lenticule shape transform is given for a cornea under a flat patient interface, with sine x =sin(x)/x. The lenticule shape transform may be given by:

$$\Delta'(\rho', \varphi) = \Delta(\rho, \varphi) \cdot \mathrm{sinc}(2\theta), \ \ \mathrm{with} \ \rho' = R \cdot \theta$$

**Curved Patient Interface Examples**

If a patient interface is not flat, but curved in any way, the translation calculations can be changed to accommodate for the curve. Below are theoretical calculations which may be used to determine the translation of points between a lenticule in a natural free state cornea, and one applanated by a curved interface.

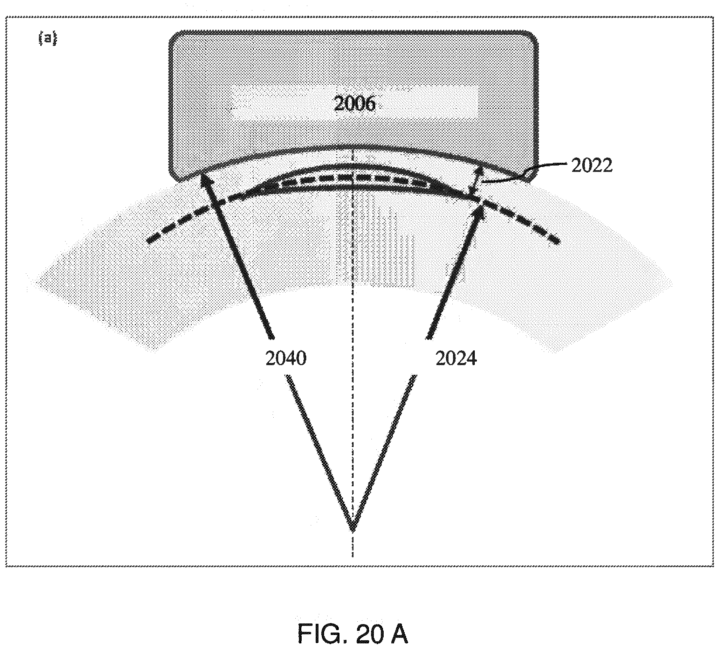

FIG. 20 A shows a two dimensional side view of a cornea under a curved patient interface 2006. It is clear that the interface 2006 does not have a flat surface like that shown in FIG. 19. Instead, the interface is curved in a concave manner. Although docking such a curved patient interface may distort the cornea less than the flat interface, some changes may still occur under applanation. The cut depth of lenticule central plane from the cornea surface 2022 is shown which could be referred to as H. The distance from the focal point to the lenticule center line 2024 may be found using the formula R'=R_PI-H, where R_PI is the distance from the focal point to the interface and cornea 2006.

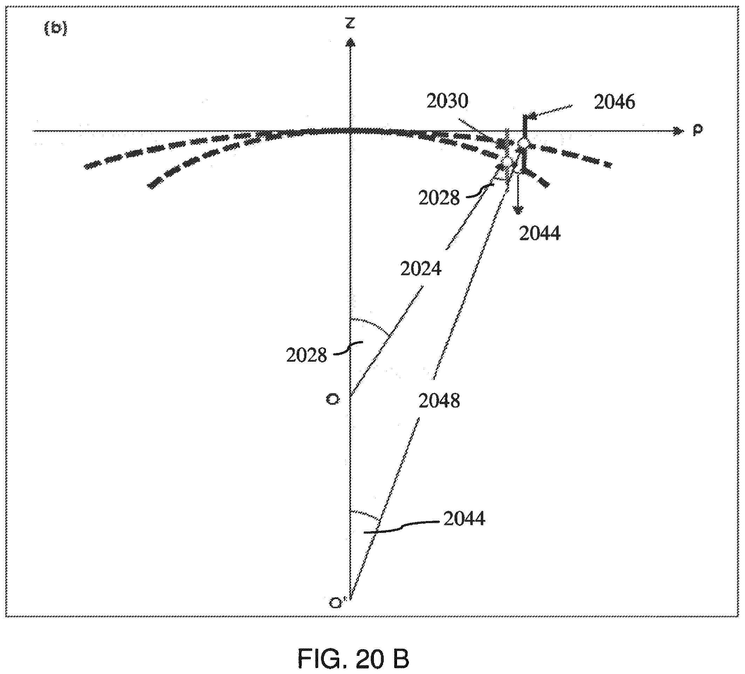

**FIG. 20B** shows an example of definitions of parameters shown in FIG. 20A. In FIG. 20B, the definitions in the diagram include: Where 2040 is RPI which is a radius of curvature of the curved patient interface. And 2048 is R' = RPI-H which is a radius of curvature of the center plane of lenticule under curved patient interface. And 2022 is H which is the cut depth of lenticule central plane. And 2042 is ($\rho'$, $\varphi$) which is a location of interest under curved patient interface. And 2044 is $\theta'$ which is an angle subtended at location $\rho'$ under curved patient interface. And 2046 is $\Delta'(\rho', \varphi)$ which is a Z-difference of lenticule surfaces at location ($\rho'$, $\varphi$) under curved patient interface. And 2024 is R=Ra-H which is a Radius of curvature of the lenticular central plane, before applanation. And 2028 is $\theta$ which is an Angle subtended by location of interest relative to Z-axis. And 2030 is $\Delta(\rho, \varphi)$ which is a Z-difference between lenticule surfaces at ($\rho$, $\varphi$) before applanation.

Thus, the lenticule shape transform under curved patient interface is given by:

$$\Delta'(\rho', \varphi) = \Delta(\rho, \varphi) \cdot \frac{\mathrm{sinc}(2\theta)}{\mathrm{sinc}(2\theta')} , \qquad \text{with} \quad \theta' = \frac{R \cdot \theta}{R'} \text{ and } \rho' = R' \cdot \sin\theta'$$

It should be noted that any kind of ultrashort laser may be used to treat the eye and incise the cornea. Examples of ultrashort lasers include but are not limited to picosecond, femtosecond, or nanosecond lasers. In some examples embodiments, the ultrashort pulsed laser has a pulse width between 10 fs and 5 ns. In some example embodiments, the ultrashort pulsed laser has a wavelength spectrum centered at between 320 nm and 1200 nm. In some example embodiments, the ultrashort pulsed laser has a pulse width between 80 fs and 250 fs. In some example embodiments, the ultrashort pulsed laser has a wavelength spectrum centered at, or has a center mass, between 1020 nm and 1070 nm and/or more specifically between 1025 nm and 1065 nm. Different embodiments may also include lasers with different profiles such as a Beckman peak or single peak.

These systems and methods could be used to treat any kind of refractive error such as myopia with or without astigmatism, hyperopia with or without astigmatism, and mixed astigmatism. Any kind of cutting profiles can be incised using these systems and methods including but not limited to lenticules, PRK removal of corneal layers, or other shapes. Transition profiles may be incised as well such as extending the top and bottom of a lenticule to aid in removal of the lenticule. Other transition profiles may include an internal side cut. In some examples, the side incision has an arc length between 1mm and 10mm.

In some examples, an apex of the lenticule and an apex of the cornea may be between 60 $\mu$m and 200 $\mu$m. In some examples, the lenticule has a lateral diameter between 4 mm and 8 mm.

The use of the terms "a" and "an" and "the" and similar referents in the context of describing the embodiments (especially in the context of the following claims) are to be construed to cover both the singular and the plural, unless otherwise indicated herein or clearly contradicted by context. The terms "comprising," "having," "including," and "containing" are to be construed as open-ended terms (*i.e.*, meaning "including, but not limited to,") unless otherwise noted. The term "connected" is to be construed as partly or wholly contained within, attached to, or joined together, even if there is something intervening. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g.*, "such as") provided herein, is intended merely to better illuminate embodiments and does not pose a limitation on the scope of the embodiments unless otherwise claimed. No language in the specification should be construed as indicating any non-claimed element as essential to the practice of the embodiments.

As disclosed herein, features may be implemented via computer-hardware, software and/or firmware. For example, the systems disclosed herein may be embodied in various forms including, for example, a data processor, such as a computer that also includes a database, digital electronic circuitry, firmware, software, computer networks, services, or in combinations of them. Further, while some of the disclosed implementations describe specific hardware components, systems and methods may be implemented with any combination of hardware, software and/or firmware. Moreover, the above-noted features may be implemented in various environments. Such environments and related applications may be specially constructed for performing the various routines, processes and/or operations or they may include a general-purpose computer or computing platform selectively activated or reconfigured by code to provide the necessary functionality. The processes disclosed herein are not inherently related to any particular computer, network, architecture, environment, or other apparatus, and may be implemented by a suitable combination of hardware, software, and/or firmware. For example, various general-purpose machines may be used with, or it may be more convenient to construct a specialized apparatus or system to perform the required methods and techniques.

Aspects of the method and system described herein, such as the logic, may be implemented as functionality programmed into any of a variety of circuitry, including programmable logic devices ("PLDs"), such as field programmable gate arrays ("FPGAs"), programmable array logic ("PAL") devices, electrically programmable logic and memory devices and standard cell-based devices, as well as application specific integrated circuits. Some other possibilities for implementing aspects include: memory devices, microcontrollers with memory (such as 1PROM), embedded microprocessors, firmware, software, etc. Furthermore, aspects may be embodied in microprocessors having software-based circuit emulation, discrete logic (sequential and combinatorial), custom devices, fuzzy (neural) logic, quantum devices, and hybrids of any of the above device types. The underlying device technologies may be provided in a variety of component types, e.g., metal-oxide semiconductor field-effect transistor ("MOSFET") technologies like complementary metal-oxide semiconductor ("CMOS"), bipolar technologies like emitter-coupled logic ("ECL"), polymer technologies (e.g., silicon-conjugated polymer and metal-conjugated polymer-metal structures), mixed analog and digital, and so on.

It should also be noted that the various logic and/or functions disclosed herein may be enabled using any number of combinations of hardware, firmware, and/or as data and/or instructions embodied in various machine-readable or computer-readable media, in terms of their behavioral, register transfer, logic component, and/or other characteristics. Computer-readable media in which such formatted data and/or instructions may be embodied include, but are not limited to, non-volatile storage media in various forms (e.g., optical, magnetic or semiconductor storage media) and carrier waves that may be used to transfer such formatted data and/or instructions through wireless, optical, or wired signaling media or any combination thereof. Examples of transfers of such formatted data and/or instructions by carrier waves include, but are not limited to, transfers (uploads, downloads, e-mail, etc.) over the Internet and/or other computer networks via one or more data transfer protocols (e.g., HTTP, FTP, SMTP, and so on).

Unless the context clearly requires otherwise, throughout the description and the claims, the words "comprise," "comprising," and the like are to be construed in an inclusive sense as opposed to an exclusive or exhaustive sense; that is to say, in a sense of "including, but not limited to." Words using the singular or plural number also include the plural or singular number respectively. Additionally, the words "herein," "hereunder," "above," "below," and words of similar import refer to this application as a whole and not to any particular portions of this application. When the word "or" is used in reference to a list of two or more items, that word covers all of the following interpretations of the word: any of the items in the list, all of the items in the list and any combination of the items in the list.

The invention is defined in the claims. Other embodiments or examples not falling within the scope of the claims do not represent the invention. Various surgical and therapeutic methods are described herein. Whilst no claim is directed to these methods *per se,* the laser system of the invention is capable of being used and is intended to be used in such methods.

**Claims**

1.  A system for performing ophthalmic surgery, comprising:

    a wavefront aberrometer, configured to obtain a wavefront map of an eye in its natural state to measure a refractive error;
    a first iris imager, in communication with the wavefront aberrometer, configured to obtain a first iris image of the eye in its natural state;
    a laser surgery device including,

        a patient interface configured to dock to the eye;
        a second iris imager, configured to obtain a second iris image for the docked eye;
        a computer processor and memory in communication with the wavefront aberrometer, configured to:

            determine a cutting profile to cut in a cornea of the eye in its natural state, based on the wavefront aberrometer measurement; wherein the cutting profile for the eye in its natural state includes a lenticule shape comprising a top lenticular incision and a bottom lenticular incision; and
            determine a translation of the cutting profile for the docked eye using the cutting profile, the iris image for the eye in its natural state and the iris image for the docked eye; and

an ultrashort pulsed laser in communication with the computer processor;

**characterized in that:**
a portion of the patient interface that contacts the patient cornea comprises a liquid interface;

the computer processor and memory are configured to:

determine a first translation of the cutting profile based on an estimated perturbation of the eye by docking to the patient interface; and
determine a second translation of the cutting profile for the docked eye using the iris image for the docked eye compared to the iris image of the eye in its natural state, wherein the second translation of the cutting profile accounts for iris distortion in the second iris image for the docked eye; and

the ultrashort pulsed laser is configured to incise the bottom lenticular incision and the top lenticular incision in the cornea on the docked eye based on the first translation of the cutting profile and the second translation of the cutting profile.

2. The system of claim 1, wherein the computer processor and memory are configured to:

determine if the lenticule shape in the cornea is less than 40 $\mu$m thick; and
if so, to add thickness to the cutting profile, the added thickness imparting no refractive error correction.

3. The system of claim 1 or claim 2, wherein the refractive correction includes high order aberration correction.

4. The system of any preceding claim, wherein the translation of the cutting profile includes compensation for cyclotorsion rotation.

5. The system of any preceding claim, wherein the ultrashort pulsed laser is a femtosecond pulsed laser.

6. The system of any preceding claim, wherein the ultrashort pulsed laser has a pulse width between 10 fs and 5 ns.

7. The system of any preceding claim, wherein the iris image is taken with at least one of white light illumination or infrared illumination.

8. The system of any preceding claim, wherein the ultrashort pulsed laser has a pulse width between 80 fs and 250 fs.

9. The system of any preceding claim wherein the first translation of the cutting profile and the second translation of the cutting profile comprise a lenticule shape.

10. The system of claim 9, wherein the first translation of the cutting profile and the second translation of the cutting profile include at least one of a transition profile or an entry incision for removal of a corneal lenticule.

11. The system of claim 10, wherein the transition profile is a continuation of the bottom lenticular incision and the top lenticular incision.

12. The system of claim 10 or claim 11, wherein the entry incision has an arc length between 1mm and 10mm.

13. The system of claim 1, wherein the ultrashort pulsed laser has a wavelength spectrum centered at between 320 nm and 1200 nm.

14. The system of claim 13, wherein the ultrashort pulsed laser has a wavelength spectrum centered at between 1020 nm and 1070 nm.

**Patentansprüche**

1. System zum Durchführen von Augenchirurgie, umfassend:

ein Wellenfrontaberrometer, ausgelegt zum Erhalten einer Wellenfrontkarte eines Auges in seinem natürlichen Zustand, um einen Refraktionsfehler zu messen;

eine erste Iris-Abbildungseinrichtung, in Kommunikation mit dem Wellenfrontaberrometer, ausgelegt zum Erhalten eines ersten Irisbilds des Auges in seinem natürlichen Zustand;

eine Laserchirurgieeinrichtung, beinhaltend

eine Patientenschnittstelle, ausgelegt zum Andocken an dem Auge;

eine zweite Iris-Abbildungseinrichtung, ausgelegt zum Erhalten eines zweiten Irisbilds für das angedockte Auge;

einen Computerprozessor und einen Arbeitsspeicher in Kommunikation mit dem Wellenfrontaberrometer, ausgelegt zum:

Bestimmen eines Schnittprofils zum Schneiden in eine Hornhaut des Auges in seinem natürlichen Zustand auf der Grundlage der Wellenfrontaberrometermessung, wobei das Schnittprofil für das Auge in seinem natürlichen Zustand eine Linsengestalt beinhaltet, die einen oberen Linseneinschnitt und einen unteren Linseneinschnitt umfasst; und

Bestimmen einer Übertragung des Schnittprofils für das angedockte Auge unter Verwendung des Schnittprofils, des Irisbilds für das Auge in seinem natürlichen Zustand und des Irisbilds für das angedockte Auge; und

einen Ultra-Kurzpuls-Laser in Kommunikation mit dem Computerprozessor;

**dadurch gekennzeichnet, dass:**

ein Anteil der Patientenschnittstelle, der die Patientenhornhaut kontaktiert, eine Flüssigkeitsschnittstelle ist;

der Computerprozessor und der Arbeitsspeicher ausgelegt sind zum:

Bestimmen einer ersten Übertragung des Schnittprofils auf der Grundlage einer geschätzten Störung des Auges durch Andocken an die Patientenschnittstelle; und

Bestimmen einer zweiten Übertragung des Schnittprofils für das angedockte Auge unter Verwendung des Irisbilds für das angedockte Auge im Vergleich mit dem Irisbild des Auges in seinem natürlichen Zustand, wobei die zweite Übertragung des Schnittprofils Irisverzerrung in dem zweiten Irisbild für das angedockte Auge berücksichtigt; und

wobei der Ultra-Kurzpuls-Laser ausgelegt ist zum Einschneiden des unteren Linseneinschnitts und des oberen Linseneinschnitts in die Hornhaut in dem angedockten Auge auf der Grundlage der ersten Übertragung des Schnittprofils und der zweiten Übertragung des Schnittprofils.

2. System nach Anspruch 1, wobei der Computerprozessor und der Arbeitsspeicher ausgelegt sind zum:

Bestimmen, ob die Linsengestalt in der Hornhaut weniger als 40 $\mu$m dick ist; und

falls dem so ist, Hinzufügen von Dicke zu dem Schnittprofil, wobei die hinzugefügte Dicke keine Refraktionsfehlerkorrektur vermittelt.

3. System nach Anspruch 1 oder Anspruch 2, wobei die Refraktionskorrektur Aberrationskorrektur höherer Ordnung beinhaltet.

4. System nach einem vorhergehenden Anspruch, wobei die Übertragung des Schnittprofils Kompensation für Zyklotorsionsdrehung beinhaltet.

5. System nach einem vorhergehenden Anspruch, wobei der Ultra-Kurzpuls-Laser ein Femtosekunden-Pulslaser ist.

6. System nach einem vorhergehenden Anspruch, wobei der Ultra-Kurzpuls-Laser eine Pulsbreite zwischen 10 fs und 5 ns aufweist.

7. System nach einem vorhergehenden Anspruch, wobei das Irisbild mit Weißlichtbeleuchtung und/oder Infrarotbeleuchtung aufgenommen wird.

8. System nach einem vorhergehenden Anspruch, wobei der Ultra-Kurzpuls-Laser eine Pulsbreite zwischen 80 fs und 250 fs aufweist.

9. System nach einem vorhergehenden Anspruch, wobei die erste Übertragung des Schnittprofils und die zweite Übertragung des Schnittprofils eine Linsengestalt einschließen.

10. System nach Anspruch 9, wobei die erste Übertragung des Schnittprofils und die zweite Übertragung des Schnittprofils ein Übergangsprofil und/oder einen Eintrittseinschnitt zum Entfernen einer Hornhautlinse beinhalten.

11. System nach Anspruch 10, wobei das Übergangsprofil eine Fortsetzung des unteren Linseneinschnitts und des oberen Linseneinschnitts ist.

12. System nach Anspruch 10 oder Anspruch 11, wobei der Eintrittseinschnitt eine Bogenlänge zwischen 1 mm und 10 mm aufweist.

13. System nach Anspruch 1, wobei der Ultra-Kurzpuls-Laser ein Wellenlängenspektrum aufweist, das zwischen 320 nm und 1200 nm zentriert ist.

14. System nach Anspruch 13, wobei der Ultra-Kurzpuls-Laser ein Wellenlängenspektrum aufweist, das zwischen 1020 nm und 1070 nm zentriert ist.

**Revendications**

1. Système pour réaliser une chirurgie ophtalmique, comprenant :

   un aberromètre à front d'onde, configuré pour obtenir une carte du front d'onde d'un œil dans son état naturel afin de mesurer une erreur de réfraction ;
   un premier imageur d'iris, en communication avec l'aberromètre à front d'onde, configuré pour obtenir une première image d'iris de l'œil dans son état naturel ;
   un dispositif de chirurgie au laser comprenant

   une interface patient configurée pour s'arrimer à l'œil ;
   un second imageur d'iris, configuré pour obtenir une seconde image d'iris pour l'œil arrimé ;
   un processeur informatique et une mémoire en communication avec l'aberromètre à front d'onde, configurés pour :

   déterminer un profil de coupe pour couper la cornée de l'œil dans son état naturel, sur la base de la mesure de l'aberromètre à front d'onde ; le profil de coupe pour l'œil dans son état naturel comprenant une forme de lenticule comprenant une incision lenticulaire supérieure et une incision lenticulaire inférieure ; et
   déterminer une translation du profil de coupe pour l'œil arrimé à l'aide du profil de coupe, de l'image de l'iris pour l'œil à l'état naturel et de l'image de l'iris pour l'œil arrimé ; et

   un laser à impulsions ultracourtes en communication avec le processeur informatique ;

   **caractérisé en ce que**
   une partie de l'interface patient qui entre en contact avec la cornée du patient comprend une interface liquide ;
   le processeur informatique et la mémoire sont configurés pour :

   déterminer une première translation du profil de coupe en fonction d'une perturbation estimée de l'œil par l'arrimage à l'interface patient ; et
   déterminer une deuxième translation du profil de coupe pour l'œil arrimé en utilisant l'image de l'iris de l'œil arrimé par rapport à l'image de l'iris de l'œil dans son état naturel, la deuxième translation du profil de coupe tenant compte de la distorsion de l'iris dans la deuxième image de l'iris de l'œil arrimé ; et

   le laser à impulsions ultracourtes étant configuré pour inciser l'incision lenticulaire inférieure et l'incision lenticulaire supérieure dans la cornée de l'œil arrimé en fonction de la première translation du profil de coupe et de la seconde translation du profil de coupe.

2. Système selon la revendication 1, le processeur informatique et la mémoire étant configurés pour :

déterminer si la forme de lenticule dans la cornée a une épaisseur inférieure à 40 um ; et

dans l'affirmative, ajouter de l'épaisseur au profil de coupe, l'épaisseur ajoutée n'entraînant aucune correction de l'erreur de réfraction.

3. Système selon la revendication 1 ou selon la revendication 2, la correction réfractive comprenant une correction d'aberration d'ordre élevé.

4. Système selon n'importe quelle revendication précédente, la translation du profil de coupe comprenant la compensation de la rotation de la cyclotorsion.

5. Système selon n'importe quelle revendication précédente, le laser à impulsions ultracourtes étant un laser à impulsions femtosecondes.

6. Système selon n'importe quelle revendication précédente, le laser à impulsions ultracourtes ayant une largeur d'impulsion comprise entre 10 fs et 5 ns.

7. Système selon n'importe quelle revendication précédente, l'image de l'iris étant prise avec au moins l'une d'un éclairage en lumière blanche ou d'un éclairage en infrarouge.

8. Système selon n'importe quelle revendication précédente, le laser à impulsions ultracourtes ayant une largeur d'impulsion comprise entre 80 fs et 250 fs.

9. Système selon n'importe quelle revendication précédente, la première translation du profil de coupe et la seconde translation du profil de coupe ayant une forme de lenticule.

10. Système selon la revendication 9, la première translation du profil de coupe et la deuxième translation du profil de coupe comprenant au moins un profil de transition ou une incision d'entrée pour le retrait d'un lenticule cornéen.

11. Système selon la revendication 10, le profil de transition étant une continuation de l'incision lenticulaire inférieure et de l'incision lenticulaire supérieure.

12. Système selon la revendication 10 ou selon la revendication 11, l'incision d'entrée ayant une longueur d'arc comprise entre 1 mm et 10 mm.

13. Système selon la revendication 1, le laser à impulsions ultracourtes ayant un spectre de longueur d'onde centré entre 320 nm et 1200 nm.

14. Système selon la revendication 13, le laser à impulsions ultracourtes ayant un spectre de longueur d'onde centré entre 1020 nm et 1070 nm.

Conventional Lenticular Cut via
Scanning Single Spot

Fig. 1

**Fig. 2**

Fig. 3

**Fig. 4**

EP 3 782 591 B1

Fast-Scan
(L≈1mm
v≈25m/sec
f≈8000Hz)

Slow Sweep
(v <0.1m/sec)

Fig. 5

Fig. 6

Fast Scan
710

L

B

δ, Deviation from the Intended Incision

720 Intended Spherical Dissection Surface

R

A

O

**Fig. 7**

**Fig. 8**

```
                              ┌─────────────┐
                              │    Start    │
                              └──────┬──────┘
                                     │
                                     ▼
                        ┌────────────────────────────┐
900                     │ Perform pre-op measurements │
                        └────────────┬───────────────┘
  910                                │
                                     ▼
                        ┌────────────────────────────┐
                        │   Calculate curvature of cut │
                        └────────────┬───────────────┘
  920                                │
                                     ▼
                        ┌────────────────────────────┐
                        │   Calculate diameter of cut  │
                        └────────────┬───────────────┘
  930                                │
                                     ▼
                        ┌────────────────────────────┐
                        │       Perform side cut       │
                        └────────────┬───────────────┘
  940                                │
                                     ▼
                        ┌────────────────────────────┐
                        │   Perform bottom surface     │
                        │        dissection            │
                        └────────────┬───────────────┘
  950                                │
                                     ▼
                        ┌────────────────────────────┐
                        │ Perform top surface dissection │
                        └────────────┬───────────────┘
  960                                │
                                     ▼
                        ┌────────────────────────────┐
                        │   Extract lenticular tissue  │
                        └────────────┬───────────────┘
  970                                │
                                     ▼
                              ┌─────────────┐
                              │     End     │
                              └─────────────┘
```

Fig. 9

**Fig. 10**

**Fig. 11**

EP 3 782 591 B1

**Fig. 12**

Fig. 13

Fig. 14

EP 3 782 591 B1

```
                          ┌─────────┐
                          │  Start  │
                          └─────────┘
                               │
                               ▼
                    ┌──────────────────────┐
       1500   ───►  │ Obtain Wavefront Target│
                    └──────────────────────┘
      1510                     │
                               ▼
                    ┌──────────────────────┐
                    │   Obtain Femto Target │
                    └──────────────────────┘
      1520                     │
                               ▼
                    ┌──────────────────────┐
                    │   Obtain Femto Image  │
                    └──────────────────────┘
      1530                     │
      1540                     ▼
                    ┌──────────────────────┐
                    │  Perform Femto Treatment│
                    │        Planning       │
                    └──────────────────────┘
                               │
                               ▼
                    ┌──────────────────────┐
      1550          │    Perform side cut   │
                    └──────────────────────┘
                               │
                               ▼
                    ┌──────────────────────┐
                    │ Perform bottom surface│
      1560          │   concave dissection  │
                    └──────────────────────┘
                               │
                               ▼
                    ┌──────────────────────┐
                    │ Perform top surface concave│
      1570          │       dissection      │
                    └──────────────────────┘
                               │
                               ▼
                    ┌──────────────────────┐
      1580          │ Extract lenticular tissue│
                    └──────────────────────┘
                               │
                               ▼
                          ┌─────────┐
                          │   End   │
                          └─────────┘
```

**Fig. 15A**

Fig. 15B

FIG. 16A

FIG. 16B

Detector

Video Target

SLD

From Fig. 16A

16100
16166
16155
16180
16182
16186
16184
16156
16154
16152
16150
16165
16164
16166
16163
16160
16100
185
183
162
153

FIG. 17

EP 3 782 591 B1

1801 — Align instrument to eye

1805 — Activate Target Fixation subsystem for Patient fixation on target

1810 — Activate wavefront subsystem to measures eyes refraction via the wavefront sensor

1815 — Moves target fixation target to optimum position and active wavefront subsystem to measure eyes refraction via wavefront sensor

1820 — Obtain iris image using Iris Imaging Subsystem while infrared lights flash

1825 — Operate z-scan device to set location at cornea, and Perform OCT Scan with OCT Subsystem

1830 — Operate z-scan device to set location at lens anterior, and Perform OCT Scan with OCT Subsystem

1835 — Operate z-scan device to set location at lens posterior, and Perform OCT Scan with OCT

1840 — Operate X-scan device and Y-scan device so no light from OCT reaches detector 141

1845 — Obtain iris image using Iris Imaging Subsystem while infrared lights flash

1850 — Obtain iris image using Iris Imaging Subsystem while CT Cone and Helmholtz lights flash

1855 — Operate z-scan device to set location at retina, and Perform OCT Scan with OCT Subsystem

1860 — Operate X-scan device and Y-scan device so no light from OCT reaches detector 141

1865 — Measure corneal topography with Corneal Topography Subsystem while CT Cone and Helmholtz lights flash

1870 — Infrared Illumination lights flash, iris image taken (for 3D model)

End

FIG. 18

FIG. 19

FIG. 20 A

FIG. 20 B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011035036 A
- US 6315413 B
- US 8260024 B
- US 4764930 A
- US 5993438 A
- US 98706911
- US 20110172649 A
- US 79845713
- US 20140104576 A
- US 84873315
- US 86539615
- US 96854915
- US 97089815 A
- US 5646791 A
- US 4665913 A
- US 4669466 A

- US 4732148 A
- US 4770172 A
- US 4773414 A
- US 5207668 A
- US 5108388 A
- US 5219343 A
- US 5163934 A
- US 8394084 B
- US 8403921 B
- US 8690862 B
- US 8709001 B
- US 98706911 A
- US 97089815
- US 6550917 B, Neal
- US 5777719 A, Williams
- US 418841

**Non-patent literature cited in the description**

- **HAIXIA LIU ; LARRY THIBOS ; CAROLYN G. BEGLEY ; ARTHUR BRADLEY.** MEASUREMENT OF THE TIME COURSE OF OPTICAL QUALITY AND VISUAL DETERIORATION DURING TEAR BREAK-UP. *Investigative Ophthalmology & Visual Science,* June 2010, vol. 51 (6

- **KOB.** *Simultaneous Measurement of Tear Film Dynamics IOVS,* July 2010, vol. 51 (7